(19) Europäisches Patentamt
European Patent Office
Office européen des brevets

(11) Veröffentlichungsnummer : **0 453 398 A2**

(12) **EUROPÄISCHE PATENTANMELDUNG**

(21) Anmeldenummer : **91810225.2**

(22) Anmeldetag : **27.03.91**

(51) Int. Cl.[5] : **C07D 213/61,** A01N 43/40, C07D 213/38, C07D 277/32, C07D 213/89, A01N 43/78

(30) Priorität : **06.04.90 CH 1169/90**
**02.11.90 CH 3481/90**

(43) Veröffentlichungstag der Anmeldung : **23.10.91 Patentblatt 91/43**

(84) Benannte Vertragsstaaten : **AT BE CH DE DK ES FR GB GR IT LI LU NL**

(71) Anmelder : **CIBA-GEIGY AG**
**Klybeckstrasse 141**
**CH-4002 Basel (CH)**

(72) Erfinder : **Maienfisch, Peter, Dr.**
**Aegertenstrasse 21**
**CH-4118 Rodersdorf (CH)**
Erfinder : **Gsell, Laurenz, Dr.**
**Maiengasse 56**
**CH-4056 Basel (CH)**
Erfinder : **Kristiansen, Odd, Dr.**
**Delligrabenstrasse 7**
**CH-4313 Möhlin (CH)**

(54) **Nitroenamin-Derivate als Schädlingbekämpfungsmittel.**

(57) Neue Nitroenamin-Derivate der Formel I

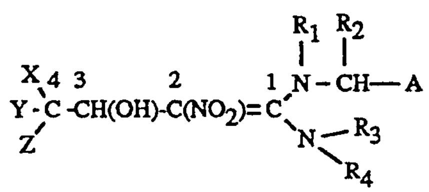

(I)

worin
$R_1$ Wasserstoff, $C_1$-$C_6$-Alkyl oder $C_3$-$C_6$-Cycloalkyl,
$R_2$ Wasserstoff oder $C_1$-$C_6$-Alkyl,
$R_3$ Wasserstoff, $C_1$-$C_6$-Alkyl oder $C_3$-$C_6$-Cycloalkyl,
$R_4$ Wasserstoff, $C_1$-$C_6$-Alkyl, $C_3$-$C_6$-Cycloalkyl oder einen Rest $-CH_2-B$, oder $R_3$ und $R_4$ gemeinsam $-(CH_2)_4-$ oder $-(CH_2)_5-$ ;
X Halogen oder $C_1$-$C_6$-Haloalkyl ;
Y und Z Halogen ;
A einen unsubstituierten oder ein- bis vierfach substituierten aromatischen oder nicht-aromatischen, monocyclischen oder bicyclischen heterocyclischen Rest, worin ein oder zwei Substituenten der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten der Gruppe $C_1$-$C_3$-Alkyl, $C_1$-$C_3$-Alkoxy und Halogen angehören ;
B Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl ; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl bedeuten, sowie deren Salze mit anorganischen Säuren ; wobei nur einer der Reste $R_1$, $R_3$ und $R_4$ für Wasserstoff stehen kann,
sowie deren Salze können als Schädlingsbekämpfungsmittel eingesetzt werden. Vorzugsweise können Insekten und Arachniden bekämpft werden.

EP 0 453 398 A2

Die vorliegende Erfindung betrifft neue Nitroenamin-Derivate, d.h. neue 1,1-Diamino-2-nitro-3-hydroxybut-1-en-Derivate, Verfahren zu ihrer Herstellung, Schädlingsbekämpfungsmittel, welche diese Verbindungen enthalten, sowie ihre Verwendung bei der Kontrolle von Schädlingen.

Diese erfindungsgemässen Nitroenamin-Derivate entsprechen der Formel I

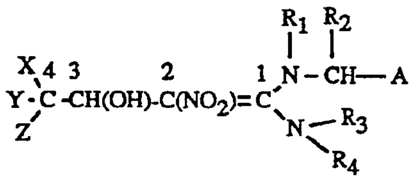

(I)

worin

$R_1$ Wasserstoff, $C_1$-$C_6$-Alkyl oder $C_3$-$C_6$-Cycloalkyl,

$R_2$ Wasserstoff oder $C_1$-$C_6$-Alkyl,

$R_3$ Wasserstoff, $C_1$-$C_6$-Alkyl oder $C_3$-$C_6$-Cycloalkyl,

$R_4$ Wasserstoff, $C_1$-$C_6$-Alkyl, $C_3$-$C_6$-Cycloalkyl oder einen Rest $-CH_2$-B, oder $R_3$ und $R_4$ gemeinsam $-(CH_2)_4$- oder $-(CH_2)_5$-;

X Halogen oder $C_1$-$C_6$-Haloalkyl;

Y und Z Halogen;

A einen unsubstituierten oder ein- bis vierfach substituierten aromatischen oder nicht-aromatischen, monocyclischen oder bicyclischen heterocyclischen Rest, worin ein oder zwei Substituenten der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten der Gruppe $C_1$-$C_3$-Alkyl, $C_1$-$C_3$-Alkoxy und Halogen angehören;

B Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl bedeuten, sowie deren Salze mit anorganischen Säuren; wobei nur einer der Reste $R_1$, $R_3$ und $R_4$ für Wasserstoff stehen kann.

Bevorzugt werden diejenigen erfindungsgemässen Verbindungen der Formel I, worin der heterocyclische Rest A ungesättigt ist und über eines seiner Ring-Kohlenstoffatome an den Rest der Verbindung der Formel I gebunden ist; diejenigen, worin der heterocyclische Rest A mindestens ein Stickstoffatom enthält; und diejenigen, worin der heterocyclische Rest A ein bis drei Heteroatome aus der Reihe Sauerstoff, Schwefel und Stickstoff enthält, von denen höchstens eines ein Sauerstoff- oder Schwefelatom ist.

Die in der Definition des heterocyclischen Restes A umfassten Ringsysteme enthalten somit als Ringglied mindestens ein Heteroatom, das heisst mindestens eines der den zugrundeliegenden ringförmigen Grundkörper bildenden Atome ist von Kohlenstoff verschieden. Grundsätzlich sind alle Atome des periodischen Systems der Elemente befähigt, als Ringglieder zu fungieren, sofern sie mindestens zwei kovalente Bindungen bilden können. Der heterocyclische Rest ist dabei bevorzugt ungesättigt und über ein Kohlenstoffatom - Ringglied an den Grundkörper des Nitroenamins der Formel I gebunden. Ungesättigte Ringsysteme der Definition A somit enthalten eine oder mehrere Doppelbindungen. Vorzugsweise sind derartige Ringsysteme mehrfach ungesättigt und weisen im allgemeinen aromatischen Charakter auf. Bevorzugt sind Ringsysteme unter der Definition von A, worin der heterocyclische Rest A ein bis drei Heteroatome aus der Reihe Sauerstoff, Schwefel und Stickstoff enthält, wovon stets ein Heteroatom Stickstoff ist und höchstens ein Sauerstoffatom oder Schwefelatom enthalten ist. Beispiele für erfindungsgemässe heterocyclische Reste der Definition A finden sich in der Gruppe von Grundkörpern der folgenden Strukturen:

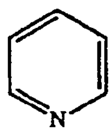 , 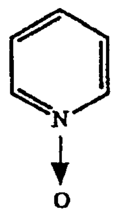 , 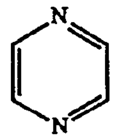 , 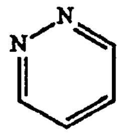 , 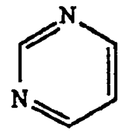 , 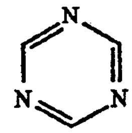 , 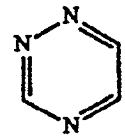 ,

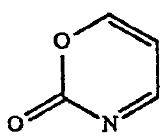 , 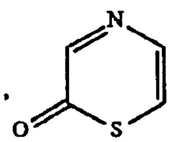 , 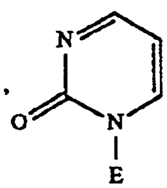 , 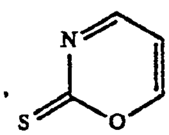 , 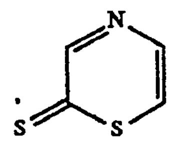 , 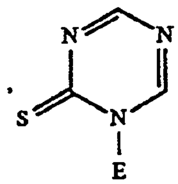 ,

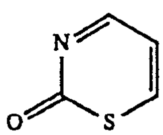 , 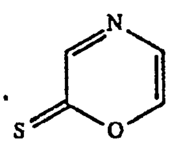 , 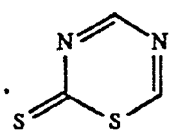 , 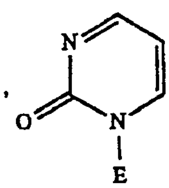 , 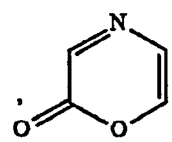 ,

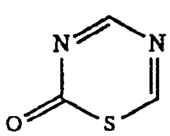 , 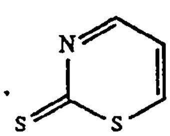 , 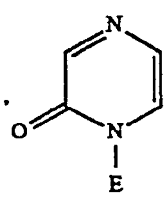 , 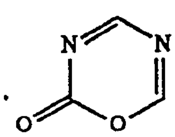 , 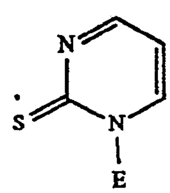 , 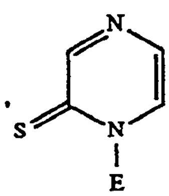 ,

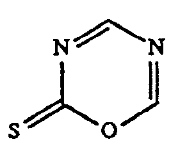 , 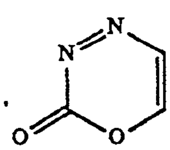 , 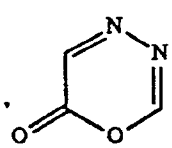 , 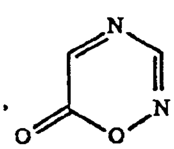 , 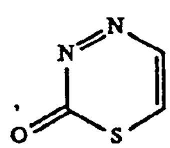 ,

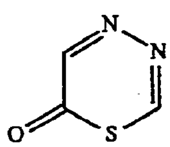 , 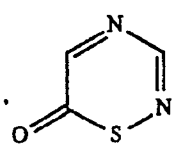 , 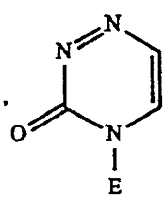 , 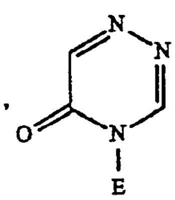 , 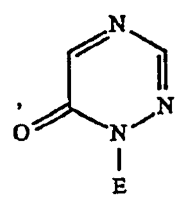 ,

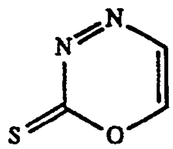 , 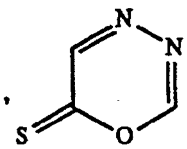 , 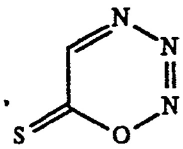 , 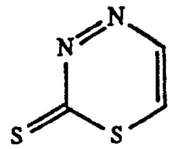 , 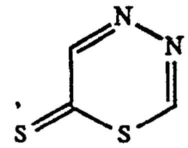 ,

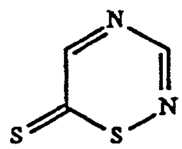 , 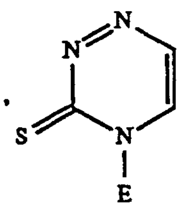 , 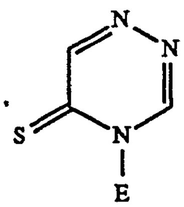 , 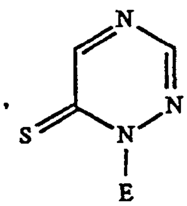 , 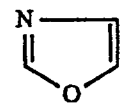 ,

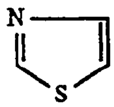 , 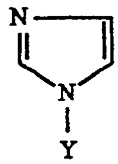 , 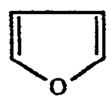 , 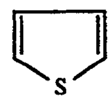 ,

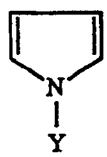 , 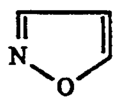 , 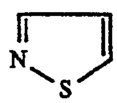 , 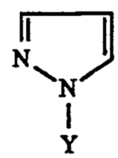 ,

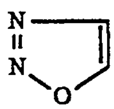 , 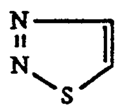 , 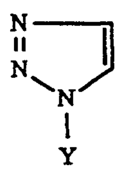 , 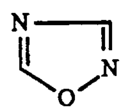 , 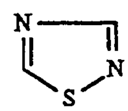 ,

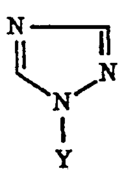 , 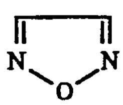 , 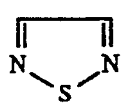 , 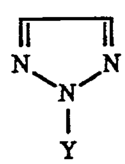 , 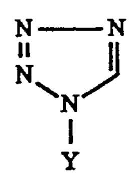 ,

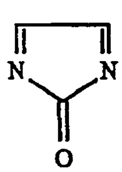 , 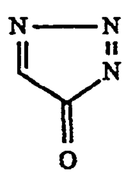 , 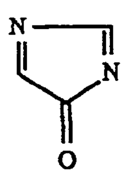 , 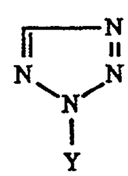 , 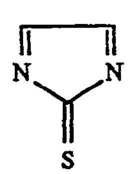 ,

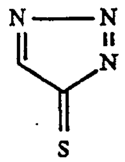 , 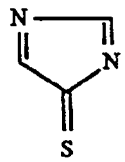 , 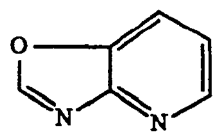 , 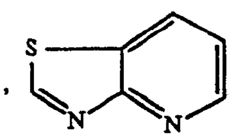 , 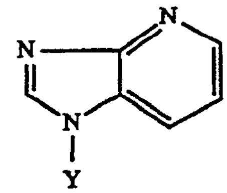 ,

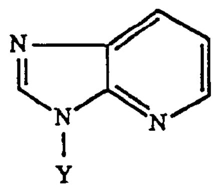 , 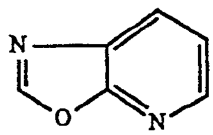 , 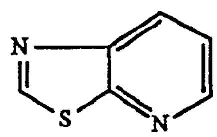 , 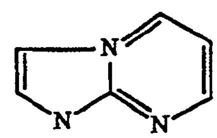 ,

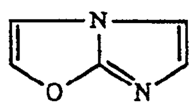 , 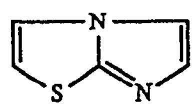 , 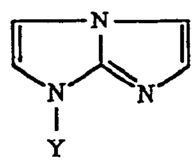 ,

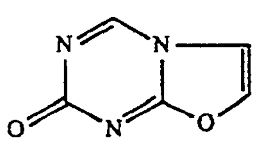 , 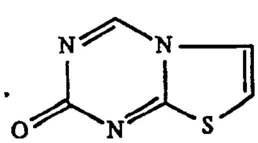 , 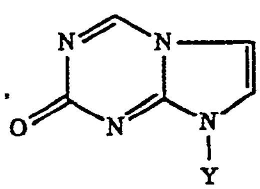 , 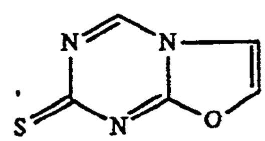 ,

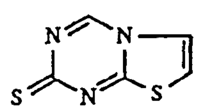 und 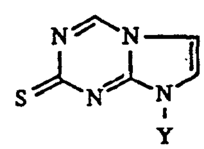 ,

wobei ein Rest gemäss den obigen Formeln unsubstituiert ist oder je nach den Substitutionsmöglichkeiten des betreffenden Ringsystems bis zu vier der unter Formel I definierten Substituenten tragen kann und E für $C_1$-$C_3$-Alkyl und

Y für Wasserstoff, $C_1$-$C_3$-Alkyl oder Cyclopropyl stehen. Bevorzugt sind diese Heterocyclen A unsubstituiert oder sie tragen ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy. Ganz besonders bevorzugte Heterocyclen A sind Pyridylreste oder Thiazolylreste, wie zum Beispiel 3-Pyridyl, 2-Halogenpyrid-5-yl, 2,3-Dihalogenpyrid-5-yl, 2-Halogenthiazol-4-yl, 1-Oxopyrid-3-yl, 1-Oxo-2-halogenpyrid-5-yl, 1-Oxo-2,3-dihalogenpyrid-5-yl.

Weiterhin hervorzuheben sind solche erfindungsgemässe Verbindungen der Formel I, die dadurch gekennzeichnet sind, dass der Rest B für einen Phenyl-, Pyridyl- oder Thiazolylrest steht, welcher unsubstituiert oder durch ein bis zwei Reste aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiert ist.

Aufgrund ihrer biologischen Wirkung bevorzugt werden solche Verbindungen der Formel I, worin $R_1$ und $R_3$ Wasserstoff, Methyl, Äthyl oder Cyclopropyl bedeuten; worin $R_2$ Wasserstoff bedeutet; worin $R_4$ Wasserstoff oder Methyl bedeutet; worin $R_1$ und $R_3$ unabhängig voneinander Wasserstoff, Methyl, Äthyl oder Cyclopropyl, $R_2$ Wasserstoff, $R_4$ Wasserstoff oder Methyl, A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiertes Pyridyl, 1-Oxopyridyl oder Thiazolyl bedeuten; worin X Fluor, Chlor, Brom oder $C_1$-$C_3$-Haloalkyl mit 1 bis 7 Fluoratomen und Y und Z unabhängig voneinander Fluor, Chlor oder Brom bedeuten, wobei insbesondere X, Y und Z unabhängig voneinander Fluor, Chlor oder Brom, und speziell X, Y und Z Chlor bedeuten; worin $R_1$ und $R_3$ Methyl, $R_2$ und $R_4$ Wasserstoff; und X, Y und Z unabhängig voneinander Fluor, Chlor oder Brom bedeuten; worin A für Pyridyl oder durch ein oder zwei Chloratome substituiertes Pyridyl steht; oder worin A für Thiazolyl oder durch ein Chloratom substituiertes Thiazolyl, vorzugsweise 1,3-Thiazol-5-yl, steht.

Von speziellem Interesse wegen ihrer guten pestiziden Eigenschaften sind solche erfindungsgemässen Verbindungen der Formel I, worin X Fluor oder Brom, vorzugsweise Fluor, und Y und Z Halogen, vorzugsweise Fluor oder Chlor-, solche, worin X $C_1$-$C_8$-Halogenalkyl mit 4 bis 13, vorzugsweise 2 bis 13, Halogenatomen; sowie solche, worin X Trifluormethyl oder einen der Reste -$C_2F_5$ oder -$C_3F_7$ bedeuten.

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor, Chlor und Brom bevorzugt sind. Halogen ist dabei als selbständiger Substituent oder als Teil eines Substituenten zu verstehen wie im Halogenalkyl, Halogenalkylthio, Halogenalkoxy, Halogencycloalkyl, Halogenalkenyl, Halogenalkinyl, Halogenallyloxy oder Halogenallylthio. Die als Substituenten in Betracht kommenden Alkyl-, Alkylthio-, Alkenyl-, Alkinyl- und Alkoxyreste können geradkettig oder verzweigt sein. Als Beispiele solcher Alkyle seien Methyl, Aethyl, Propyl, Isopropyl, Butyl, i-Butyl, sek.-Butyl oder tert.-Butyl genannt. Als geeignete Alkoxyreste seien unter anderem genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio. Sind die als Substituenten in Betracht kommenden Alkyl-, Alkoxy-, Alkenyl-, Alkinyl- oder Cycloalkylgruppen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das ein- bis dreifach durch Fluor, Chlor und/oder Brom substituierte Methyl wie beispielsweise $CHF_2$ oder $CF_3$; das ein- bis fünffach durch Fluor, Chlor und/oder Brom substituierte Aethyl wie zum Beispiel $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie beispielsweise $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie zum Beispiel $CF(CF_3)CHFCF_3$ oder $CH_2(CF_2)_2CF_3$; 2-Chlorcyclopropyl oder 2,2-Difluorcyclopropyl; 2,2-Difluorvinyl, 2,2-Dichlorvinyl, 2-Chlorallyl, 2,3-Dichlorvinyl oder 2,3-Dibromvinyl.

Sind die definierten Alkyl-, Alkoxy- oder Cycloalkylgruppen durch andere Substituenten substituiert, so können sie ein- oder mehrfach durch den gleichen oder verschiedene der aufgezählten Substituenten substituiert sein. Vorzugsweise sind in den substituierten Gruppen ein oder zwei weitere Substituenten vorhanden. Bei den als Substituenten in Betracht kommenden Cycloalkylresten handelt es sich beispielsweise um Cyclopropyl, Cyclobutyl, Cyclopentyl oder Cyclohexyl. Alkenyl- und Alkinylgruppen enthalten eine ungesättigte Kohlenstoff-Kohlenstoff-Bindung. Typische Vertreter sind Allyl, Methallyl oder Propargyl, aber auch Vinyl und Äthinyl. Die Doppel- oder Dreifachbindungen in Allyloxy, Propargyloxy, Allylthio oder Propargylthio sind von der Verknüpfungsstelle zum Heteroatom (O oder S) vorzugsweise durch ein gesättigtes Kohlenstoffatom getrennt.

Es sind bereits heterocyclische Verbindungen, welche eine Nitroenaminstruktur enthalten, aus EP-A-192 060, EP-A-302 389 und EP-A-302 833 als Insektizide bekannt; die biologischen Eigenschaften dieser Verbindungen vermögen jedoch bei der Schädlingsbekämpfung nicht voll zu befriedigen. Strukturell zeichnen sich die erfindungsgemässen Verbindungen der Formel I gegenüber den vorstehend genannten bekannten Nitroenaminen insbesondere durch das Vorliegen der speziellen 3,4-Substituenten in der n-But-1-en-Kette aus.

Die Verbindungen der erfindungsgemässen Formel I schliessen auch die Salze mit agrochemisch verträglichen anorganischen Säuren ein. Beispiele solcher Säuren sind Salzsäure, Bromwasserstoffsäure, Schwefelsäure, Phosphorsäure und Salpetersäure sowie Säuren mit gleichem Zentralatom und höherer oder niedrigerer Oxidationsstufen wie Perchlorsäure, salpetrige Säure oder phosphorige Säure.

Die erfindungsgemässen Verbindungen der Formel I können als Doppelbindungsisomere z.B. cis-, trans-Isomere, bzw. als Stereoisomere am C-Atom 3 vorliegen. Unter den Verbindungen der Formel I sollen derartige isomere Formen sowie Gemische derselben verstanden werden.

Die erfindungsgemässen Verbindungen der Formel I können in Analogie zu bekannten Verfahren hergestellt werden. Beispielsweise erhält man die Verbindung der Formel I, indem man ein 1,1-Diamino-2-nitroäthyl-Derivat der Formel II

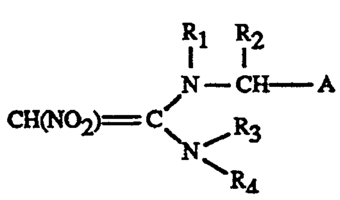

(II)

mit einer Verbindung der Formel III

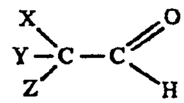

(III)

umsetzt, wobei $R_1$ bis $R_4$, A, X, Y und Z die vorstehend angegebenen Bedeutungen haben.

Die Durchführung des obigen erfindungsgemässen Verfahrens erfolgt mit Vorteil in einem inerten Lösungsmittel bei Temperaturen zwischen 0°C und + 120°C, insbesondere zwischen +20°C und +80°C. Als Lösungsmittel eignen sich in besonderer Weise: Geeignete Lösungmittel sind z.B. aromatische Kohlenwasserstoffe, wie Benzol, Toluol und Xylol; Aether, wie Tetrahydrofuran, Dioxan und Diäthyläther, halogenierte Kohlenwasserstoffe, wie Methylenchlorid, Chloroform, Tetrachlorkohlenstoff und Chlorbenzol; Alkohole, wie Methanol, Aethanol und Propanol; Ester aliphatischer Säuren, wie Aethylacetat; aliphatische Amide, wie Dimethylformamid und Dimethylacetamid; Acetonitril und andere Lösungsmittel, die die Reaktion nicht beeinträchtigen. Diese Lösungsmittel können auch als Gemische verwendet werden. Die Reaktion kann, insbesondere dann, wenn die Aldehyd-Ausgangsverbindung der Formel III in der Hydratform vorliegt, unter Zugabe eines wasserentziehenden Mittels, wie z.B. Molekularsieben, oder durch azeotrope Entfernung des entstehenden Reaktionswassers durchgeführt werden. Zweckmässigerweise sollten bei der Durchführung der Reaktion basische Bedingungen, d.h. pH-Werte >8, sowie hohe Temperaturen oberhalb etwa 100°C vermieden werden, weil die erwünschten Produkte der Formel I unter diesen Reaktionsbedingungen instabil sind.

Die Zwischenprodukte der Formeln II (1,1-Diamino-2-nitroäthylen-Derivate) und III (Haloacetaldehyde) sind bekannt oder können in Analogie zu bekannten Verfahren hergestellt werden (vgl. EP Patentanmeldung Nr. 302.389 und 302 833).

Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Die Verbindungen der Formel I sind auch zur Saatgutbehandlung (Saatbeizmittel) für die Bekämpfung insbesondere von Aphiden geeignet, wobei die sehr geringe Vogel-Toxizität der Verbindungen von grosser Bedeutung ist. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören: aus der Ordnung Lepidoptera zum Beispiel

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;

aus der Ordnung Coleoptera zum Beispiel

Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;

aus der Ordnung der Orthoptera zum Beispiel Blatta spp., Blattella spp., Gryllotalpa spp., Leucophaea maderae, Locusta spp., Periplaneta spp. und Schistocerca spp;

aus der Ordnung der Isoptera zum Beispiel

Reticulitermes spp.; aus der Ordnung der Psocoptera zum Beispiel Liposcelis spp.; aus der Ordnung der Anoplura zum Beispiel Haematopinus spp., Linognathus spp., Pediculus spp., Pemphigus spp. und Phylloxera spp.;

aus der Ordnung der Mallophaga zum Beispiel Damalinea spp. und Trichodectes spp.;

aus der Ordnung der Thysanoptera zum Beispiel

Frankliniella spp., Hercinothrips spp., Taeniothrips spp., Thrips palmi, Thrips tabaci und Scirtothrips aurantii;

aus der Ordnung der Heteroptera zum Beispiel

Cimex spp., Distantiella theobroma, Dysdercus spp., Euchistus spp., Eurygaster spp., Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insectiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage.

Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und-/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:

" 1985 International Mc Cutcheon's Emulsifiers & Detergents", Glen Rock NJ USA, 1985",
"H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 % Wirkstoff der Formel I oder Kombinationen dises Wirkstoffs mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen (% = Gewichtsprozent), wobei als Wirkstoff eine Verbindung der Formel I enthalten ist, wie z.B. die Verbindungen Nr. 1.001,

1.008, 1.009, 1.011, 1.012, 1.015, 1.028, 1.081, 1.084, 1.085, 1.101, 1.102, 1.157, 1.105, 1.109, 1.010, 1.028, 1.022:

Emulgierbare Konzentrate:

| | |
|---|---|
| Aktiver Wirkstoff: | 1 bis 20 %, bevorzugt 5 bis 10 % |
| obeflächenaktives Mittel: | 5 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspension-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| obeflächenaktives Mittel: | 1 bis 40 %, vorzugsweise 2 bis 30 % |

Benetzbare Pulver:

| | |
|---|---|
| Aktiver Wirkstoff: | 0,5 bis 90 %, vorzugsweise 1 bis 80 % |
| obeflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, Entschäumer, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Beispiel 1: Herstellung von 1-Methylamino-1-[N-(6-chlor-pyrid-3-ylmethyl)-N-methyl]-2-nitro-3-hydroxy-4,4,4-trichlor-but-1-en.

Zu einer Mischung von 2,56 g (10 mmol) 1-Methylamino-1-[N-(6-chlor-pyrid-3-ylmethyl)-N-methyl]-amino-2-nitroäthylen in 30 ml Methylenchlorid werden 1,06 ml (10,8 mmol) Chloral gegeben. Nach 2$\frac{1}{2}$-stündigem Rühren bei Raumtemperatur wird das Reaktionsgemisch filtriert, und die erhaltenen Kristalle werden mit einer grossen Menge Methylenchlorid gewaschen. Man erhält 3,81 g (94 %) der Titelverbindung der Formel

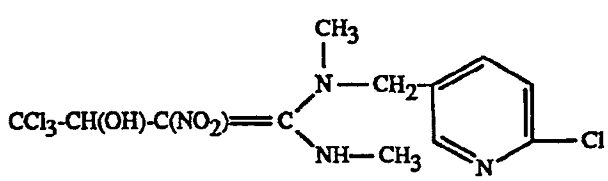

mit einem Schmelzpunkt von 130°C (Verbindung Nr. 1.008).

Beispiel 2: Herstellung von 1-Methylamino-1-[N-(6-chlor-pyrid-3-ylmethyl)-N-methyl]-2-nitro-3-hydroxy-4,4-difluor-4-chlorbut-1-en.

Zu einer Mischung von 14,4 g 1-Methylamino-1-[N-(6-chlor-pyrid-3-ylmethyl)-N-methyl]-amino-2-nitroäthylen in 150 ml Methylenchlorid werden 7,42 g Chlorofluoracetaldehyd. $H_2O$ gegeben. Nach 2-tägigem Rühren bei Raumtemperatur wird das Reaktionsgemisch filtriert, und die erhaltenen Kristalle werden mit Methylenchlorid gewaschen. Man erhält die Titelverbindung der folgenden Formel in einer Menge von 17,2 g

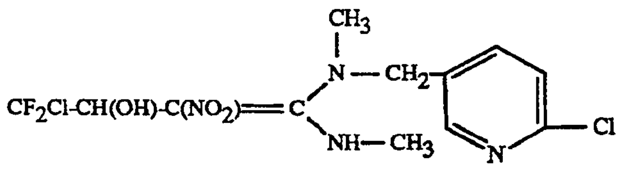

mit einem Smp. von 142-143°C (Verbindung Nr. 1.011).

Beispiel 3: Herstellung von 1-Methylamino-1-(N-(6-chlor-pyrid-3-ylmethyl)-N-methyl]-2-nitro-3-hydroxy-4,4,5,5,6,6,6-heptafluor-hex-1-en.

Zu einer Mischung von 2,56 g 1-Methylamino-1-[N-(6-chlor-pyrid-3-ylmethyl)-N-methyl]-amino-2-nitroäthylen und 3,24 g Heptafluorbutyraldehydhydrat in 50 ml Methylenchlorid werden 5 g Molekularsieb 4 Å gegeben. Nach 2-tägigem Rühren bei Raumtemperatur wird das Reaktionsgemisch filtriert und das Filtrat eingedampft. Die dabei erhaltenen Kristalle werden mit Hexan/Isopropanol (1:1) gewaschen. Man erhält 4,02 g der Titelverbindung der Formel

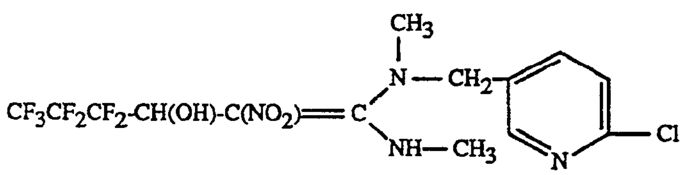

mit einem Schmelzpunkt von 93-98°C (Verbindung Nr. 1.012)

In analoger Weise wie vorstehend angegeben können die in der folgenden Tabelle 1 aufgelisteten Verbindungen der Formel Ia hergestellt werden.

Tabelle 1

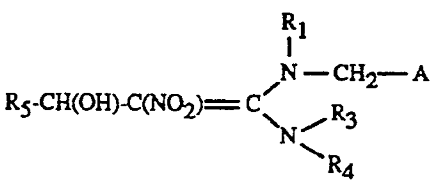

(Ia)

| Verb. Nr | A | $R_1$ | $R_3$ | $R_4$ | $R_5$ | phys. Daten |
|---|---|---|---|---|---|---|
| 1.001 | 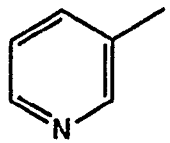 | $CH_3$ | H | $CH_3$ | $-CCl_3$ | Smp. 133°C |
| 1.002 | 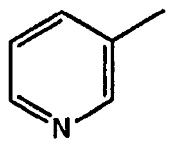 | $CH_3$ | H | $CH_3$ | $-CBr_3$ | |
| 1.003 | 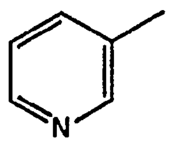 | $CH_3$ | H | $CH_3$ | $-CF_3$ | |
| 1.004 | 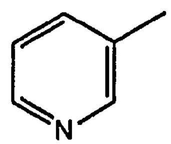 | $CH_3$ | H | $CH_3$ | $-CClF_2$ | |
| 1.005 | 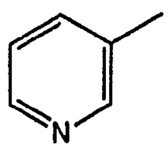 | $CH_3$ | H | $CH_3$ | $-CF_2-CF_3$ | |
| 1.006 | 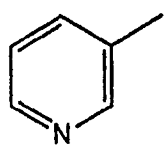 | $CH_3$ | H | $CH_3$ | $-CF_2-CF_2-CF_3$ | |

| Verb. Nr | A | $R_1$ | $R_3$ | $R_4$ | $R_5$ | phys. Daten |
|---|---|---|---|---|---|---|
| 1.007 | 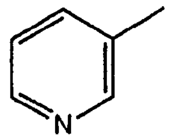 | $CH_3$ | H | $CH_3$ | $-C_7F_{15}$-n | |
| 1.008 | 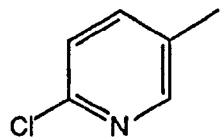 | $CH_3$ | H | $CH_3$ | $-CCl_3$ | Smp. 130°C |
| 1.009 | 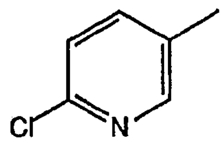 | $CH_3$ | H | $CH_3$ | $-CBr_3$ | Smp. 120-122°C |
| 1.010 | 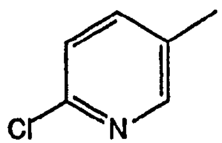 | $CH_3$ | H | $CH_3$ | $-CF_3$ | Smp. 156°C |
| 1.011 | 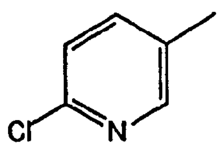 | $CH_3$ | H | $CH_3$ | $-CF_2Cl$ | Smp. 142-143°C |
| 1.012 | 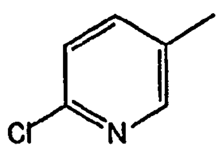 | $CH_3$ | H | $CH_3$ | $-C_3F_7$-n | Smp. 93-98°C |

| Verb. Nr | A | $R_1$ | $R_3$ | $R_4$ | $R_5$ | phys. Daten |
|---|---|---|---|---|---|---|
| 1.013 | 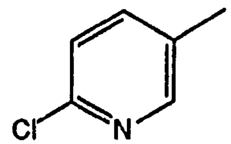 | $CH_3$ | H | $CH_3$ | $-CF_2-CF_3$ | Smp. 113-115°C |
| 1.014 | 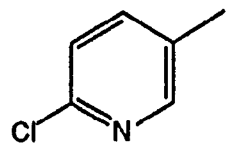 | $CH_3$ | H | $CH_3$ | $-C_7F_{15}$-n | |
| 1.015 | 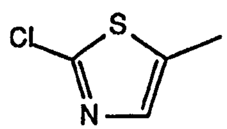 | $CH_3$ | H | $CH_3$ | $-CCl_3$ | Smp. 142-143°C |
| 1.016 | 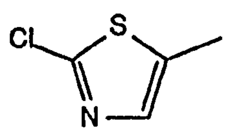 | $CH_3$ | H | $CH_3$ | $-CBr_3$ | |
| 1.017 | 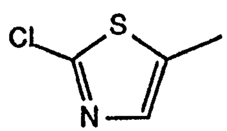 | $CH_3$ | H | $CH_3$ | $-CF_3$ | |
| 1.018 | 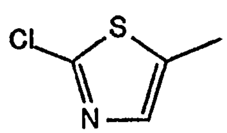 | $CH_3$ | H | $CH_3$ | $-CClF_2$ | |

| Verb. Nr | A | $R_1$ | $R_3$ | $R_4$ | $R_5$ | phys. Daten |
|---|---|---|---|---|---|---|
| 1.019 | 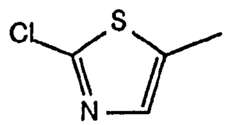 | $CH_3$ | H | $CH_3$ | $-CF_2-CF_3$ | |
| 1.020 | 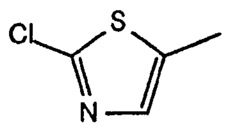 | $CH_3$ | H | $CH_3$ | $-C_3F_7$-n | |
| 1.021 | 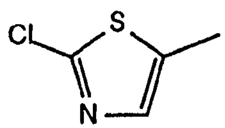 | $CH_3$ | H | $CH_3$ | $-C_7F_{15}$-n | |
| 1.022 | 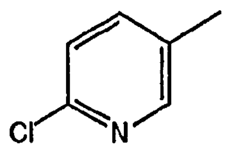 | $C_2H_5$ | H | $CH_3$ | $-CCl_3$ | Smp. 106-108°C |
| 1.023 | 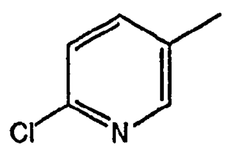 | $C_2H_5$ | H | $CH_3$ | $-CBr_3$ | |
| 1.024 | 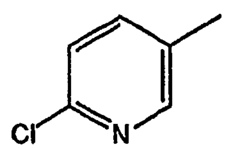 | $C_2H_5$ | H | $CH_3$ | $-CF_3$ | Smp. 174°C |

| Verb. Nr | A | $R_1$ | $R_3$ | $R_4$ | $R_5$ | phys. Daten |
|---|---|---|---|---|---|---|
| 1.025 | 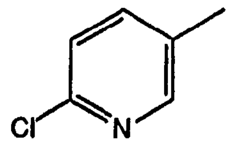 | $C_2H_5$ | H | $CH_3$ | $-CClF_2$ | Smp. 102°C |
| 1.026 | 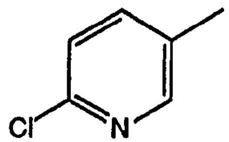 | 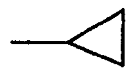 | H | $CH_3$ | $-CCl_3$ | Smp. 125-126°C |
| 1.027 | 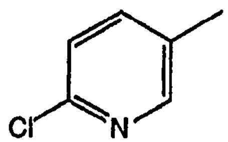 | 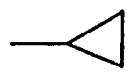 | H | $CH_3$ | $-CF_3$ | |
| 1.028 | 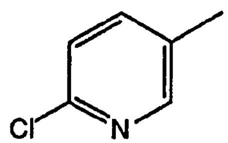 | 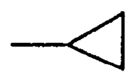 | H | $CH_3$ | $-CClF_2$ | Smp. 142-143°C |
| 1.029 | 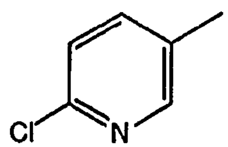 | 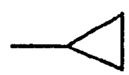 | H | $CH_3$ | $-CBr_3$ | |
| 1.030 | 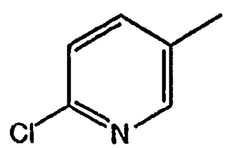 | $CH_3$ | H | $C_2H_5$ | $-CCl_3$ | |

| Verb. Nr | A | $R_1$ | $R_3$ | $R_4$ | $R_5$ | phys. Daten |
|---|---|---|---|---|---|---|
| 1.031 | 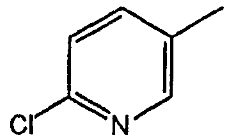 | $CH_3$ | H | $C_2H_5$ | $-CClF_2$ | |
| 1.032 | 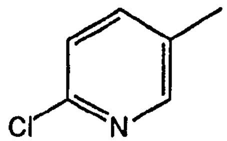 | $CH_3$ | H | $C_2H_5$ | $-CF_3$ | |
| 1.033 | 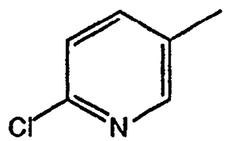 | $CH_3$ | H | 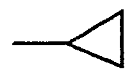 | $-CCl_3$ | |
| 1.034 | 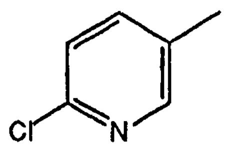 | $CH_3$ | H | 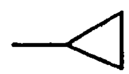 | $-CClF_2$ | |
| 1.035 | 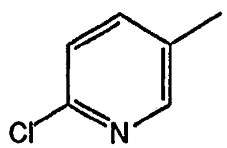 | $CH_3$ | H | 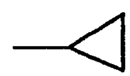 | $-CF_3$ | |
| 1.036 | 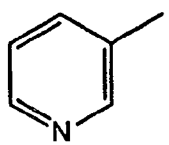 | H | $CH_3$ | $CH_3$ | $-CCl_3$ | |

| Verb. Nr | A | $R_1$ | $R_3$ | $R_4$ | $R_5$ | phys. Daten |
|---|---|---|---|---|---|---|
| 1.037 | 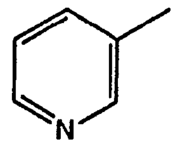 | H | $CH_3$ | $CH_3$ | $-CClF_2$ | |
| 1.038 | 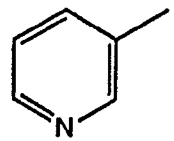 | H | $CH_3$ | $CH_3$ | $-CF_3$ | |
| 1.039 | 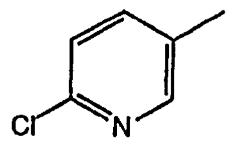 | H | $CH_3$ | $CH_3$ | $-CCl_3$ | Smp. 120°C |
| 1.040 | 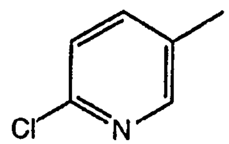 | H | $CH_3$ | $CH_3$ | $-CF_3$ | |
| 1.041 | 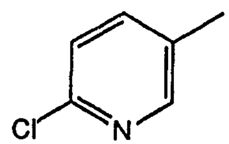 | H | $CH_3$ | $CH_3$ | $-CClF_2$ | |
| 1.042 | 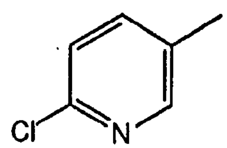 | H | $CH_3$ | $CH_3$ | $-CBr_3$ | |

| Verb. Nr | A | $R_1$ | $R_3$ | $R_4$ | $R_5$ | phys. Daten |
|---|---|---|---|---|---|---|
| 1.043 | 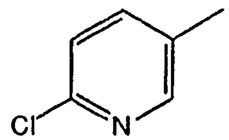 | H | $CH_3$ | $CH_3$ | $-CF_2CF_3$ | |
| 1.044 | 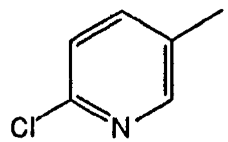 | H | $CH_3$ | $CH_3$ | $-C_3F_7$-n | |
| 1.045 | 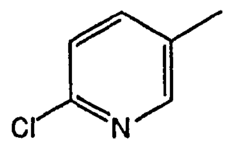 | H | $CH_3$ | $CH_3$ | $-C_7F_{15}$-n | |
| 1.046 | 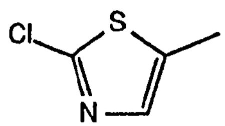 | H | $CH_3$ | $CH_3$ | $-CCl_3$ | |
| 1.047 | 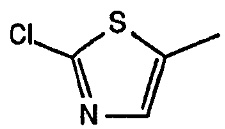 | H | $CH_3$ | $CH_3$ | $-CF_3$ | |
| 1.048 | 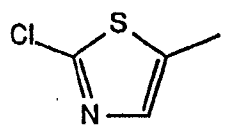 | H | $CH_3$ | $CH_3$ | $-CClF_2$ | |

| Verb. Nr | A | $R_1$ | $R_3$ | $R_4$ | $R_5$ | phys. Daten |
|---|---|---|---|---|---|---|
| 1.049 | 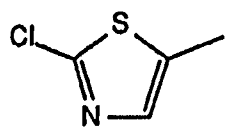 | H | $CH_3$ | $CH_3$ | $-CF_2CF_3$ | |
| 1.050 | 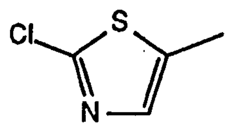 | H | $CH_3$ | $CH_3$ | $-C_3F_7$-n | |
| 1.051 | 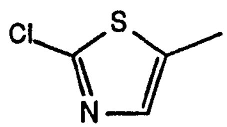 | H | $CH_3$ | $CH_3$ | $-C_7F_{15}$-n | |
| 1.052 | 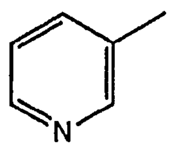 | $C_2H_5$ | H | $CH_3$ | $-CCl_3$ | |
| 1.053 | 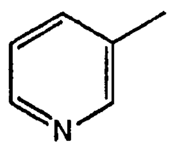 | 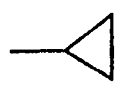 | H | $CH_3$ | $-CCl_3$ | |
| 1.054 | 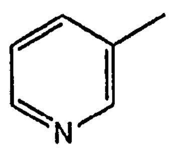 | $CH_3$ | H | $C_2H_5$ | $-CCl_3$ | |

| Verb. Nr | A | $R_1$ | $R_3$ | $R_4$ | $R_5$ | phys. Daten |
|---|---|---|---|---|---|---|
| 1.055 | 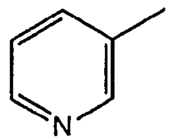 | $CH_3$ | H | 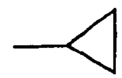 | $-CCl_3$ | |
| 1.056 | 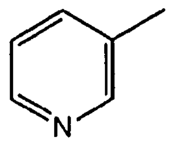 | $CH_3$ | H | $C_3H_7$-n | $-CCl_3$ | |
| 1.057 | 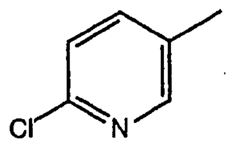 | $CH_3$ | H | $CH_2-C_6H_5$ | $-CCl_3$ | |
| 1.058 | 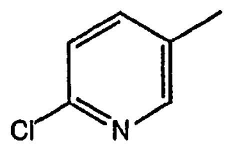 | $CH_3$ | H | $CH_2-C_6H_4-NO_2$-(3) | $-CCl_3$ | |
| 1.059 | 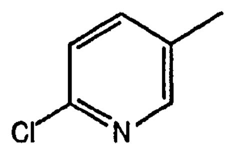 | $CH_3$ | H | $CH_2-C_6H_4$-CN-(3) | $-CCl_3$ | |
| 1.060 | 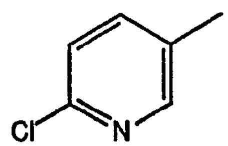 | $CH_3$ | H | $CH_2$ 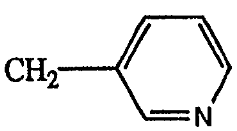 | $-CCl_3$ | |

| Verb. Nr | A | $R_1$ | $R_3$ | $R_4$ | $R_5$ | phys. Daten |
|---|---|---|---|---|---|---|
| 1.061 | 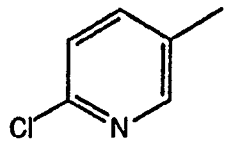 | $CH_3$ | H | 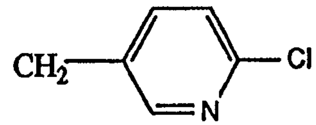 | $-CCl_3$ | |
| 1.062 | 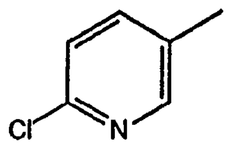 | $CH_3$ | H | $C_3H_7$-n | $-CCl_3$ | |
| 1.063 | 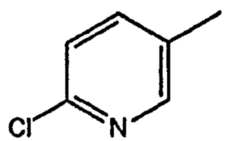 | $CH_3$ | H | $C_4H_9$-n | $-CCl_3$ | |
| 1.064 | 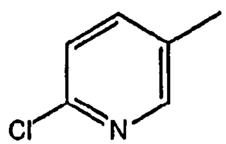 | H | $CH_3$ | $CH_2C_6H_5$ | $-CCl_3$ | |
| 1.065 | 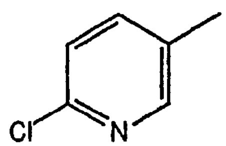 | H | $CH_3$ | $C_2H_5$ | $-CCl_3$ | |
| 1.066 | 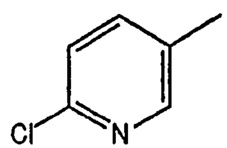 | H | $C_2H_5$ | $C_2H_5$ | $-CCl_3$ | |

| Verb. Nr | A | $R_1$ | $R_3$ | $R_4$ | $R_5$ | phys. Daten |
|---|---|---|---|---|---|---|
| 1.067 | 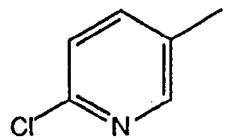 | H | $-CH_2-(CH_2)_2-CH_2-$ | | $-CCl_3$ | |
| 1.068 | 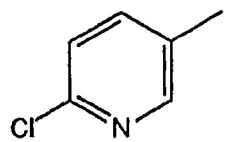 | H | $-CH_2-(CH_2)_3-CH_2-$ | | $-CCl_3$ | |
| 1.069 | 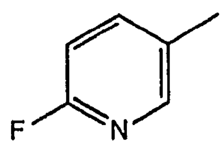 | $CH_3$ | H | $CH_3$ | $-CCl_3$ | |
| 1.070 | 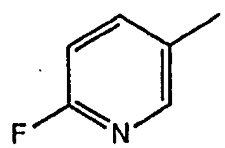 | H | $CH_3$ | $CH_3$ | $-CCl_3$ | |
| 1.071 | 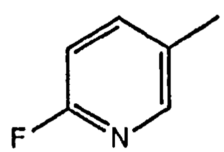 | $C_2H_5$ | H | $CH_3$ | $-CCl_3$ | |
| 1.072 | 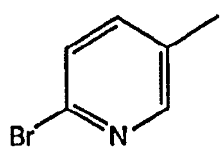 | $CH_3$ | H | $CH_3$ | $-CCl_3$ | |

| Verb. Nr | A | $R_1$ | $R_3$ | $R_4$ | $R_5$ | phys. Daten |
|---|---|---|---|---|---|---|
| 1.073 | 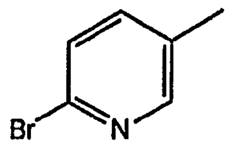 | H | $CH_3$ | $CH_3$ | $-CCl_3$ | |
| 1.074 | 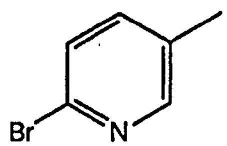 | $C_2H_5$ | H | $CH_3$ | $-CCl_3$ | |
| 1.075 | 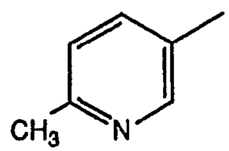 | $CH_3$ | H | $CH_3$ | $-CCl_3$ | |
| 1.076 | 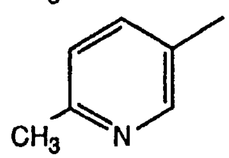 | $C_2H_5$ | H | $CH_3$ | $-CCl_3$ | |
| 1.077 | 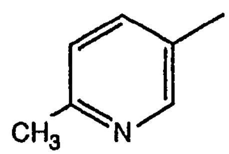 | H | $CH_3$ | $CH_3$ | $-CCl_3$ | |
| 1.078 | 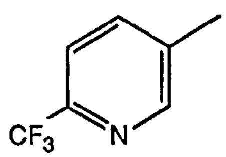 | $CH_3$ | H | $CH_3$ | $-CCl_3$ | |

| Verb. Nr | A | $R_1$ | $R_3$ | $R_4$ | $R_5$ | phys. Daten |
|---|---|---|---|---|---|---|
| 1.079 | 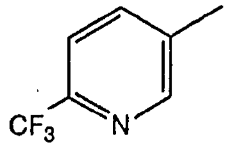 | $C_2H_5$ | H | $CH_3$ | $-CCl_3$ | |
| 1.080 | 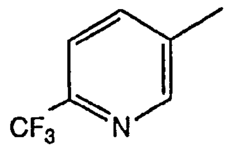 | H | H | $CH_3$ | $-CCl_3$ | |
| 1.081 | 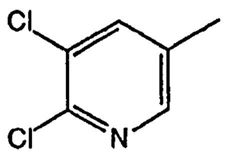 | $CH_3$ | H | $CH_3$ | $-CCl_3$ | Smp. 155-157°C |
| 1.082 | 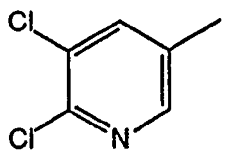 | $C_2H_5$ | H | $CH_3$ | $-CCl_3$ | |
| 1.083 | 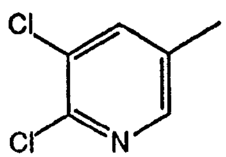 | H | $CH_3$ | $CH_3$ | $-CCl_3$ | |
| 1.084 | 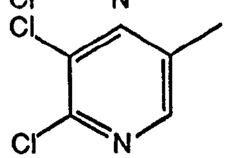 | 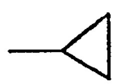 | H | $CH_3$ | $-CCl_3$ | Smp. 120,5-121,5°C |

| Verb. Nr | A | $R_1$ | $R_3$ | $R_4$ | $R_5$ | phys. Daten |
|---|---|---|---|---|---|---|
| 1.085 | 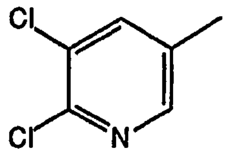 | $CH_3$ | H | $C_2H_5$ | $-CCl_3$ | Smp. 126,5-127,5°C |
| 1.086 | 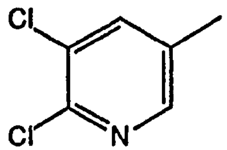 | $CH_3$ | H | 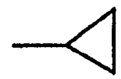 | $-CCl_3$ | |
| 1.087 | 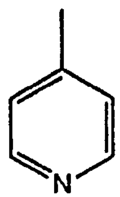 | $CH_3$ | H | $CH_3$ | $-CCl_3$ | |
| 1.088 | 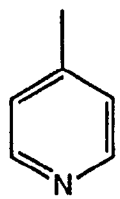 | H | $CH_3$ | $CH_3$ | $-CCl_3$ | |
| 1.089 | 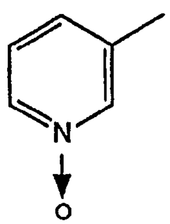 | $CH_3$ | H | $CH_3$ | $-CCl_3$ | |

| Verb. Nr | A | $R_1$ | $R_3$ | $R_4$ | $R_5$ | phys. Daten |
|---|---|---|---|---|---|---|
| 1.090 | 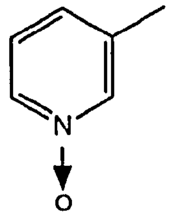 | $CH_3$ | H | $CH_3$ | $-CClF_2$ | |
| 1.091 | 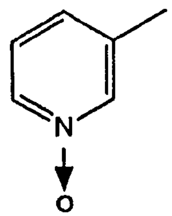 | $CH_3$ | H | $CH_3$ | $-CF_3$ | |
| 1.092 | 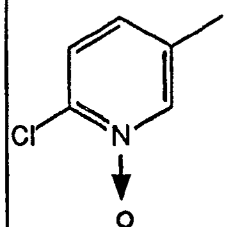 | $CH_3$ | H | $CH_3$ | $-CCl_3$ | |
| 1.093 | 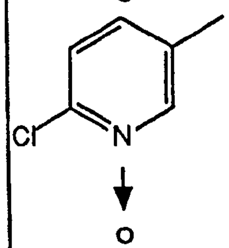 | $CH_3$ | H | $CH_3$ | $-CClF_2$ | |

| Verb. Nr | A | $R_1$ | $R_3$ | $R_4$ | $R_5$ | phys. Daten |
|---|---|---|---|---|---|---|
| 1.094 | 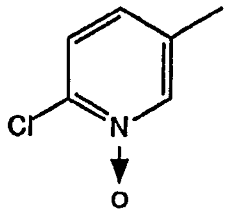 | $CH_3$ | H | $CH_3$ | $-CF_3$ | |
| 1.095 | 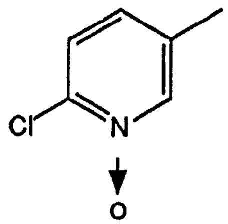 | $C_2H_5$ | H | $CH_3$ | $-CCl_3$ | |
| 1.096 | 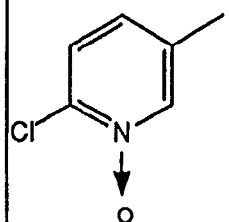 | $C_2H_5$ | H | $CH_3$ | $-CF_3$ | |
| 1.097 | 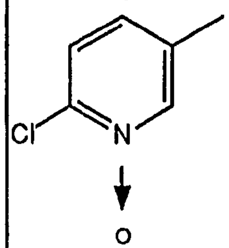 | $C_2H_5$ | H | $CH_3$ | $-CClF_2$ | |

| Verb. Nr | A | $R_1$ | $R_3$ | $R_4$ | $R_5$ | phys. Daten |
|---|---|---|---|---|---|---|
| 1.098 | 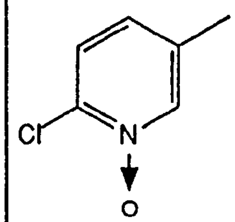 | H | $CH_3$ | $CH_3$ | $-CCl_3$ | |
| 1.099 | 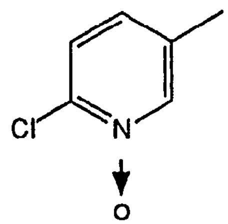 | H | $CH_3$ | $CH_3$ | $-CClF_2$ | |
| 1.100 | 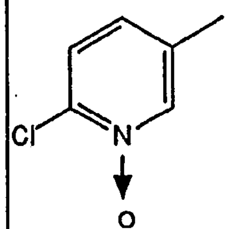 | H | $CH_3$ | $CH_3$ | $-CF_3$ | |

| Verb. Nr | A | $R_1$ | $R_3$ | $R_4$ | $R_5$ | phys. Daten |
|---|---|---|---|---|---|---|
| 1.101 | 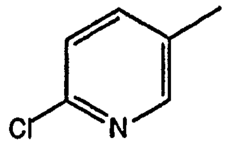 | 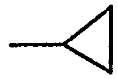 | H | $C_2H_5$ | $-CCl_3$ | Smp. 126,5-127,5°C |
| 1.102 | 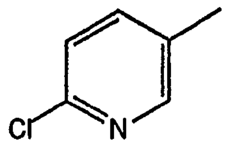 | 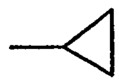 | H | 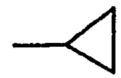 | $-CCl_3$ | Smp. 133-134°C |
| 1.103 | 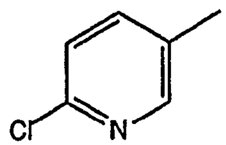 | $-C_2H_5$ | H | $-CH_3$ | $-C_3F_7$-n | Smp. 85-90°C |
| 1.104 | 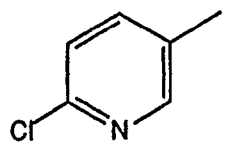 | $-C_2H_5$ | H | $-CH_3$ | $-C_7F_{15}$-n | |
| 1.105 | 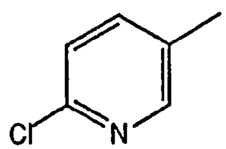 | 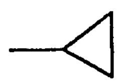 | H | $-CH_3$ | $-C_3F_7$-n | |

| Verb. Nr | A | $R_1$ | $R_3$ | $R_4$ | $R_5$ | phys. Daten |
|---|---|---|---|---|---|---|
| 1.106 | 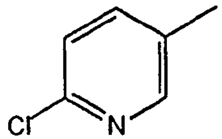 | 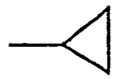 | H | $-CH_3$ | $-C_7F_{15}$-n | |
| 1.107 | 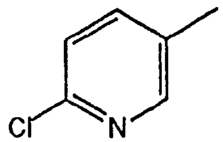 | $-CH_3$ | H | $C_2H_5$ | $-C_3F_7$-n | |
| 1.108 | 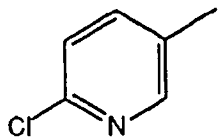 | $-CH_3$ | H | $C_2H_5$ | $-C_7F_{15}$-n | |
| 1.109 | 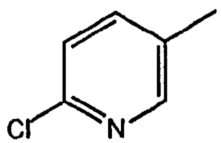 | $-CH_3$ | H | 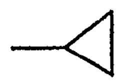 | $-C_3F_7$-n | |
| 1.110 | 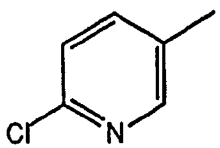 | $-CH_3$ | H | 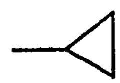 | $-C_7F_{15}$-n | |

| Verb. Nr | A | $R_1$ | $R_3$ | $R_4$ | $R_5$ | phys. Daten |
|---|---|---|---|---|---|---|
| 1.111 | 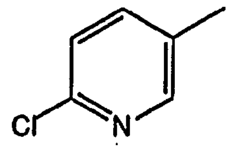 | $-C_2H_5$ | H | $-C_2H_5$ | $-CCl_3$ | |
| 1.112 | 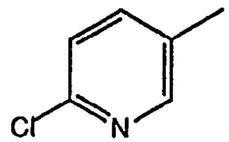 | $-C_2H_5$ | H | $-C_2H_5$ | $-CClF_2$ | |
| 1.113 | 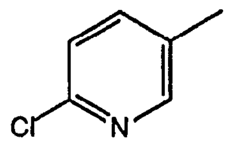 | $-C_2H_5$ | H | $-C_2H_5$ | $-CF_3$ | |
| 1.114 | 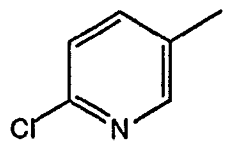 | $-C_2H_5$ | H | $-C_2H_5$ | $-C_3F_7$-n | |
| 1.115 | 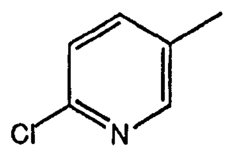 | $-C_2H_5$ | H | $-C_2H_5$ | $-C_7F_{15}$-n | |

| Verb. Nr | A | $R_1$ | $R_3$ | $R_4$ | $R_5$ | phys. Daten |
|---|---|---|---|---|---|---|
| 1.116 | 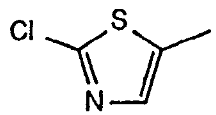 | $-C_2H_5$ | H | $-CH_3$ | $-CCl_3$ | |
| 1.117 | 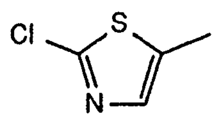 | $-C_2H_5$ | H | $-CH_3$ | $-CClF_2$ | |
| 1.118 | 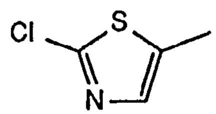 | $-C_2H_5$ | H | $-CH_3$ | $-CF_3$ | |
| 1.119 | 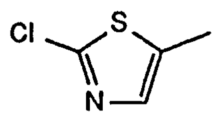 | $-C_2H_5$ | H | $-CH_3$ | $-C_3F_7$-n | |
| 1.120 | 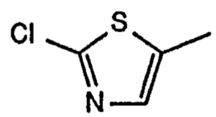 | $-C_2H_5$ | H | $-CH_3$ | $-C_7F_{15}$-n | |
| 1.121 | 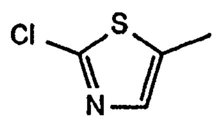 | 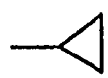 | H | $-CH_3$ | $-CCl_3$ | |
| 1.122 | 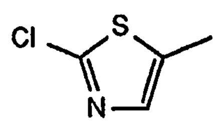 | 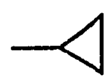 | H | $-CH_3$ | $-CClF_2$ | |
| 1.123 | 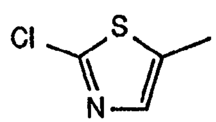 | 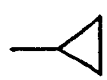 | H | $-CH_3$ | $-CF_3$ | |

| Verb. Nr | A | $R_1$ | $R_3$ | $R_4$ | $R_5$ | phys. Daten |
|---|---|---|---|---|---|---|
| 1.124 | 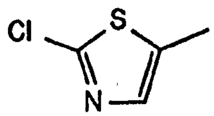 | 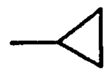 | H | $-CH_3$ | $-C_3F_7$-n | |
| 1.125 | 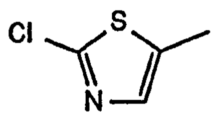 | 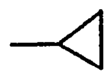 | H | $-CH_3$ | $-C_7F_{15}$-n | |
| 1.126 | 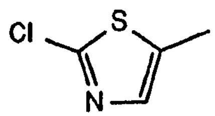 | $-CH_3$ | H | $-C_2H_5$ | $-CCl_3$ | |
| 1.127 | 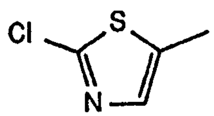 | $-CH_3$ | H | $-C_2H_5$ | $-CClF_2$ | |
| 1.128 | 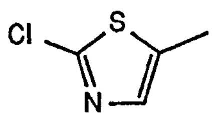 | $-CH_3$ | H | $-C_2H_5$ | $-CF_3$ | |
| 1.129 | 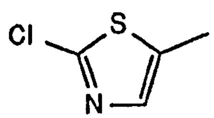 | $-CH_3$ | H | $-C_2H_5$ | $-C_3F_7$-n | |
| 1.130 | 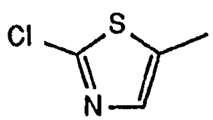 | $-CH_3$ | H | $-C_2H_5$ | $-C_7F_{15}$-n | |
| 1.131 | 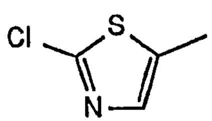 | $-C_2H_5$ | H | $-C_2H_5$ | $-CCl_3$ | |

| Verb. Nr | A | $R_1$ | $R_3$ | $R_4$ | $R_5$ | phys. Daten |
|---|---|---|---|---|---|---|
| 1.132 | 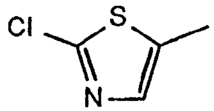 | $-C_2H_5$ | H | $-C_2H_5$ | $-CClF_2$ | |
| 1.133 | 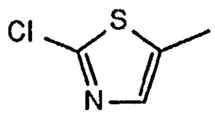 | $-C_2H_5$ | H | $-C_2H_5$ | $-CF_3$ | |
| 1.134 | 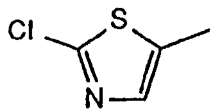 | $-C_2H_5$ | H | $-C_2H_5$ | $-C_3F_7$-n | |
| 1.135 | 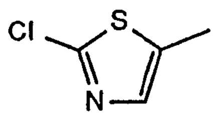 | $-C_2H_5$ | H | $-C_2H_5$ | $-C_7F_{15}$-n | |
| 1.136 | 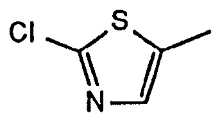 | 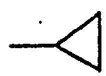 | H | $-C_2H_5$ | $-CCl_3$ | |
| 1.137 | 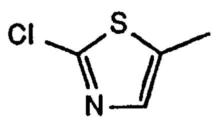 | 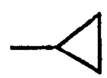 | H | $-C_2H_5$ | $-CClF_2$ | |
| 1.138 | 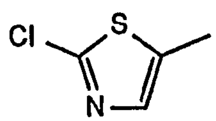 | 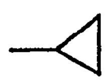 | H | $-C_2H_5$ | $-CF_3$ | |
| 1.139 | 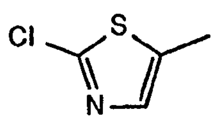 | 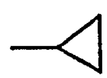 | H | $-C_2H_5$ | $-C_3F_7$-n | |

| Verb. Nr | A | $R_1$ | $R_3$ | $R_4$ | $R_5$ | phys. Daten |
|---|---|---|---|---|---|---|
| 1.140 | 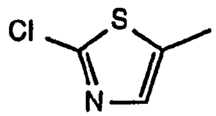 | 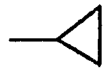 | H | $-C_2H_5$ | $-C_7F_{15}$-n | |
| 1.141 | 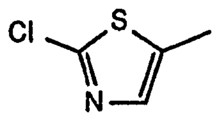 | $-CH_3$ | H | 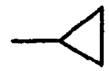 | $-CCl_3$ | |
| 1.142 | 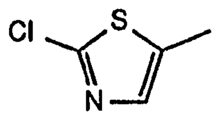 | $-CH_3$ | H | 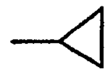 | $-CClF_2$ | |
| 1.143 | 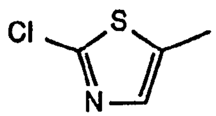 | $-CH_3$ | H | 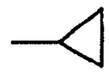 | $-CF_3$ | |
| 1.144 | 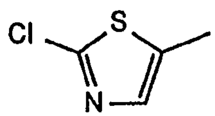 | $-CH_3$ | H | 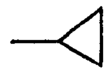 | $-C_3F_7$-n | |
| 1.145 | 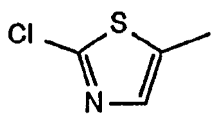 | $-CH_3$ | H | 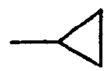 | $-C_7F_{15}$-n | |
| 1.146 | 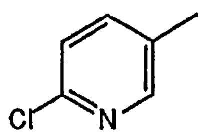 | $-CH_3$ | H | $-C_3H_7$-n | $-CClF_2$ | |

| Verb. Nr | A | $R_1$ | $R_3$ | $R_4$ | $R_5$ | phys. Daten |
|---|---|---|---|---|---|---|
| 1.147 | 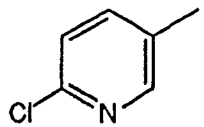 | $-CH_3$ | H | $-C_3H_7$-n | $-CF_3$ | |
| 1.148 | 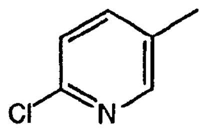 | $-CH_3$ | H | $-C_3H_7$-n | $-C_3F_7$-n | |
| 1.149 | 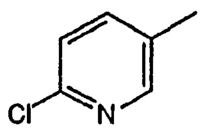 | 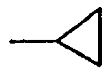 | H | $-C_2H_5$ | $-CClF_2$ | Smp. 140-141°C |
| 1.150 | 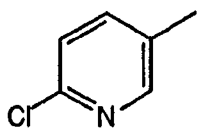 | 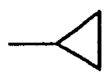 | H | $-C_2H_5$ | $-CF_3$ | |
| 1.151 | 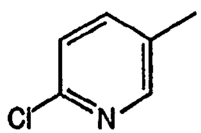 | 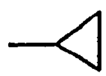 | H | $-C_2H_5$ | $-C_3F_7$-n | |
| 1.152 | 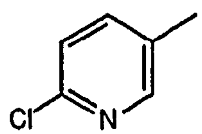 | 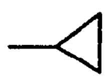 | H | $-C_2H_5$ | $-C_7F_{15}$-n | |

| Verb. Nr | A | $R_1$ | $R_3$ | $R_4$ | $R_5$ | phys. Daten |
|---|---|---|---|---|---|---|
| 1.153 | 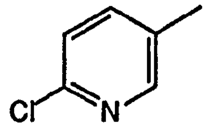 | 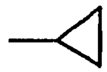 | H | 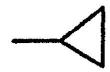 | $-CClF_2$ | Smp. 136,5-137°C |
| 1.154 | 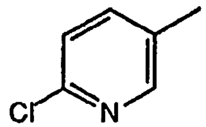 | 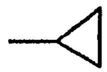 | H | 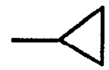 | $-CF_3$ | |
| 1.155 | 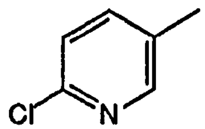 | 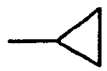 | H | 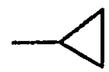 | $-C_3F_7$-n | |
| 1.156 | 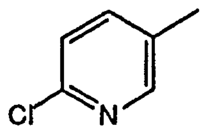 | 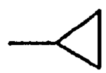 | H | 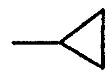 | $-C_7F_{15}$-n | |
| 1.157 | 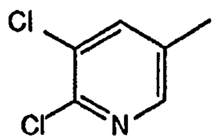 | $-CH_3$ | H | $-CH_3$ | $-CClF_2$ | Smp. 136-136,5°C |
| 1.158 | 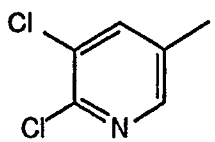 | $-CH_3$ | H | $-CH_3$ | $-CF_3$ | |

| Verb. Nr | A | $R_1$ | $R_3$ | $R_4$ | $R_5$ | phys. Daten |
|---|---|---|---|---|---|---|
| 1.159 | 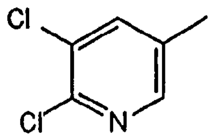 | $-CH_3$ | H | $-CH_3$ | $-C_3F_7$-n | |
| 1.160 | 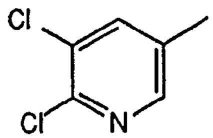 | $-CH_3$ | H | $-CH_3$ | $-C_7F_{15}$-n | |
| 1.161 | 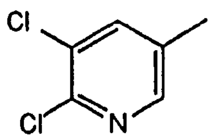 | $-C_2H_5$ | H | $-CH_3$ | $-CClF_2$ | |
| 1.162 | 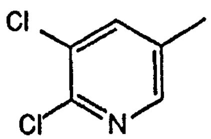 | $-C_2H_5$ | H | $-CH_3$ | $-CF_3$ | |
| 1.163 | 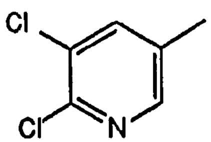 | $-C_2H_5$ | H | $-CH_3$ | $-C_3F_7$-n | |
| 1.164 | 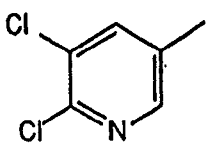 | $-C_2H_5$ | H | $-CH_3$ | $-C_7F_{15}$-n | |

| Verb. Nr | A | $R_1$ | $R_3$ | $R_4$ | $R_5$ | phys. Daten |
|---|---|---|---|---|---|---|
| 1.165 | 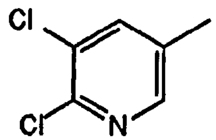 | 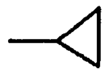 | H | $-CH_3$ | $-CClF_2$ | Smp. 140-141°C |
| 1.166 | 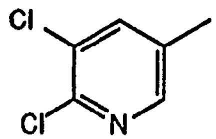 | 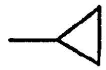 | H | $-CH_3$ | $-CF_3$ | |
| 1.167 | 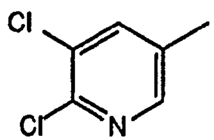 | 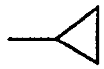 | H | $-CH_3$ | $-C_3F_7$-n | |
| 1.168 | 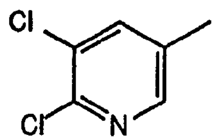 | 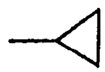 | H | $-CH_3$ | $-C_7F_{15}$-n | |
| 1.169 | 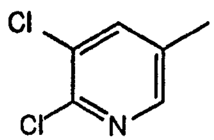 | $-CH_3$ | H | $-C_2H_5$ | $-CClF_2$ | Smp. 131-132°C |
| 1.170 | 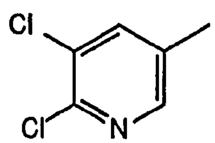 | $-CH_3$ | H | $-C_2H_5$ | $-CF_3$ | |

| Verb. Nr | A | $R_1$ | $R_3$ | $R_4$ | $R_5$ | phys. Daten |
|---|---|---|---|---|---|---|
| 1.171 | 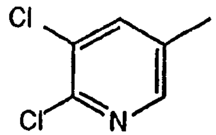 | $-CH_3$ | H | $-C_2H_5$ | $-C_3F_7$-n | |
| 1.172 | 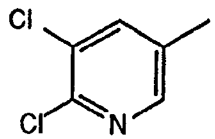 | $-CH_3$ | H | $-C_2H_5$ | $-C_7F_{15}$-n | |
| 1.173 | 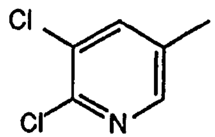 | $-CH_3$ | H | 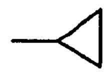 | $-CClF_2$ | |
| 1.174 | 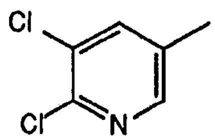 | $-CH_3$ | H | 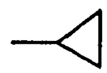 | $-CF_3$ | |
| 1.175 | 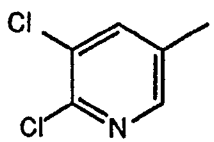 | $-CH_3$ | H | 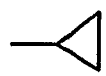 | $-C_3F_7$-n | |
| 1.176 | 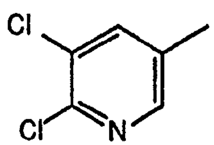 | $-CH_3$ | H | 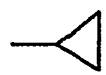 | $-C_7F_{15}$-n | |

| Verb. Nr | A | $R_1$ | $R_3$ | $R_4$ | $R_5$ | phys. Daten |
|---|---|---|---|---|---|---|
| 1.177 | 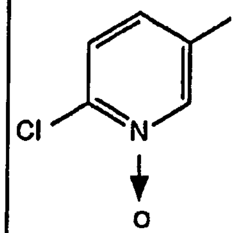 | $-CH_3$ | H | $-CH_3$ | $-C_3F_7$-n | |
| 1.178 | 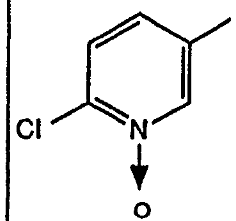 | $-CH_3$ | H | $-CH_3$ | $-C_7F_{15}$-n | |
| 1.179 | 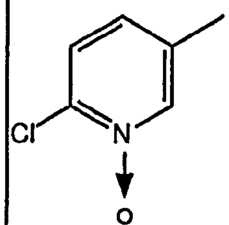 | $-C_2H_5$ | H | $-CH_3$ | $-C_3F_7$-n | |
| 1.180 | 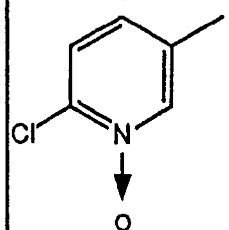 | $-C_2H_5$ | H | $-CH_3$ | $-C_7F_{15}$-n | |

| Verb. Nr | A | $R_1$ | $R_3$ | $R_4$ | $R_5$ | phys. Daten |
|---|---|---|---|---|---|---|
| 1.181 | 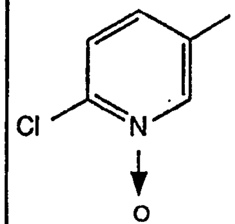 | H | $CH_3$ | $-CH_3$ | $-C_3F_7$-n | |
| 1.182 | 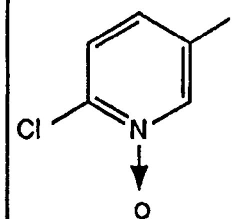 | -H | $CH_3$ | $-CH_3$ | $-C_7F_{15}$-n | |
| 1.183 | 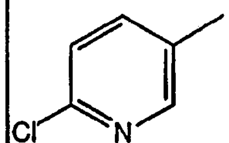 | $-CH_3$ | H | $-CH_3$ | $-CCl_2CF_3$ | Smp. 101-102°C |
| 1.184 | 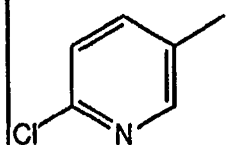 | $-C_2H_5$ | H | $-CH_3$ | $-CCl_2CF_3$ | Smp. 104-105°C |
| 1.185 | 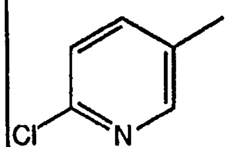 | 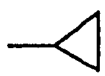 | H | $-CH_3$ | $-CCl_2CF_3$ | |

| Verb. Nr | A | $R_1$ | $R_3$ | $R_4$ | $R_5$ | phys. Daten |
|---|---|---|---|---|---|---|
| 1.186 | 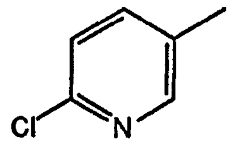 | $-CH_3$ | H | $-C_2H_5$ | $-CCl_2CF_3$ | |
| 1.187 | 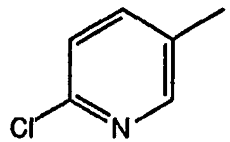 | $-CH_3$ | H | 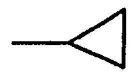 | $-CCl_2CF_3$ | |
| 1.188 | 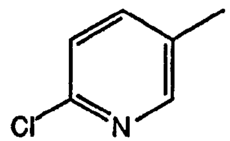 | $-C_2H_5$ | H | $-C_2H_5$ | $-CCl_2CF_3$ | |
| 1.189 | 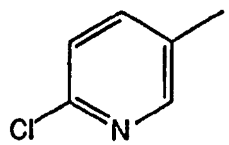 | $-C_2H_5$ | H | 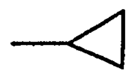 | $-CCl_2CF_3$ | |
| 1.190 | 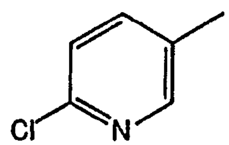 | 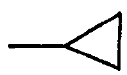 | H | $-C_2H_5$ | $-CCl_2CF_3$ | |
| 1.191 | 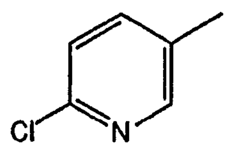 | H | $CH_3$ | $-CH_3$ | $-CCl_2CF_3$ | |

| Verb. Nr | A | $R_1$ | $R_3$ | $R_4$ | $R_5$ | phys. Daten |
|---|---|---|---|---|---|---|
| 1.192 | 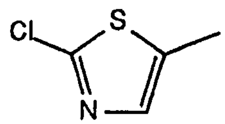 | $-CH_3$ | H | $-CH_3$ | $-CCl_2CF_3$ | |
| 1.193 | 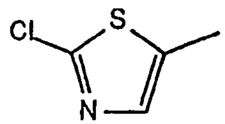 | $-C_2H_5$ | H | $-CH_3$ | $-CCl_2CF_3$ | |
| 1.194 | 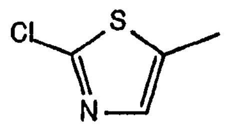 | 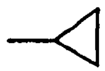 | H | $-CH_3$ | $-CCl_2CF_3$ | |
| 1.195 | 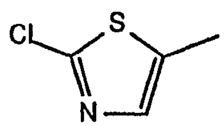 | $-CH_3$ | H | $-C_2H_5$ | $-CCl_2CF_3$ | |
| 1.196 | 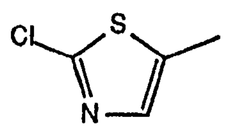 | $-CH_3$ | H | 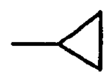 | $-CCl_2CF_3$ | |
| 1.197 | 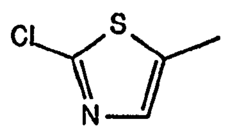 | $-C_2H_5$ | H | $-C_2H_5$ | $-CCl_2CF_3$ | |

| Verb. Nr | A | $R_1$ | $R_3$ | $R_4$ | $R_5$ | phys. Daten |
|---|---|---|---|---|---|---|
| 1.198 | 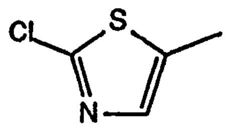 | $-C_2H_5$ | H | 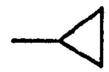 | $-CCl_2CF_3$ | |
| 1.199 | 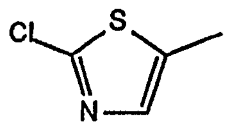 | 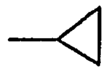 | H | $-C_2H_5$ | $-CCl_2CF_3$ | |
| 1.200 | 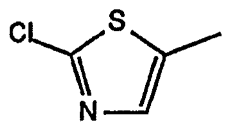 | H | $-CH_3$ | $-CH_3$ | $-CCl_2CF_3$ | |
| 1.201 | 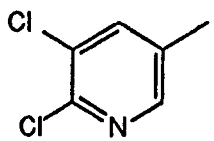 | $-CH_3$ | H | $-CH_3$ | $-CCl_2CF_3$ | |
| 1.202 | 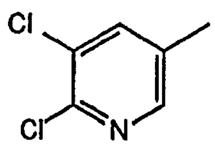 | $-C_2H_5$ | H | $-CH_3$ | $-CCl_2CF_3$ | |
| 1.203 | 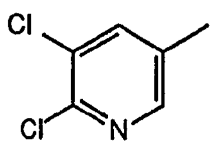 | 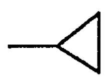 | H | $-CH_3$ | $-CCl_2CF_3$ | |

| Verb. Nr | A | $R_1$ | $R_3$ | $R_4$ | $R_5$ | phys. Daten |
|---|---|---|---|---|---|---|
| 1.204 | 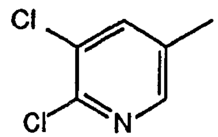 | $-CH_3$ | H | $-C_2H_5$ | $-CCl_2CF_3$ | |
| 1.205 | 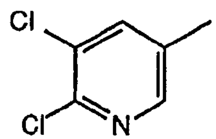 | $-CH_3$ | H | 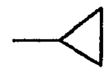 | $-CCl_2CF_3$ | |
| 1.206 | 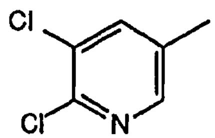 | $-C_2H_5$ | H | $-C_2H_5$ | $-CCl_2CF_3$ | |
| 1.207 | 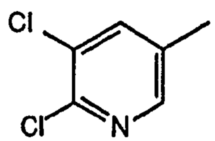 | $-C_2H_5$ | H | 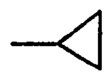 | $-CCl_2CF_3$ | |
| 1.208 | 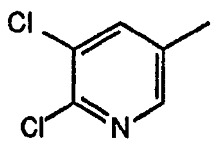 | 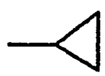 | H | $-C_2H_5$ | $-CCl_2CF_3$ | |
| 1.209 | 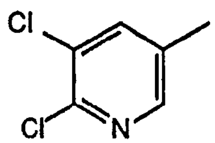 | H | $-CH_3$ | $-CH_3$ | $-CCl_2CF_3$ | |

| Verb. Nr | A | $R_1$ | $R_3$ | $R_4$ | $R_5$ | phys. Daten |
|---|---|---|---|---|---|---|
| 1.210 | 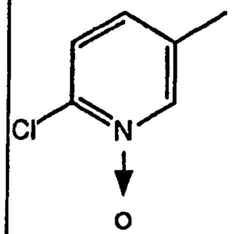 | $-CH_3$ | H | $-CH_3$ | $-CCl_2CF_3$ | |
| 1.211 | 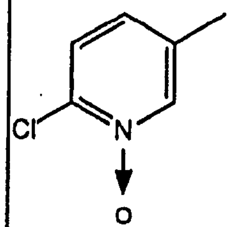 | $-C_2H_5$ | H | $-CH_3$ | $-CCl_2CF_3$ | |
| 1.212 | 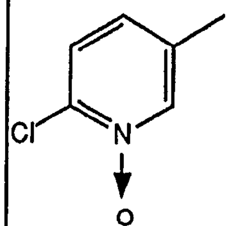 | 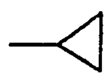 | H | $CH_3$ | $-CCl_2CF_3$ | |
| 1.213 | 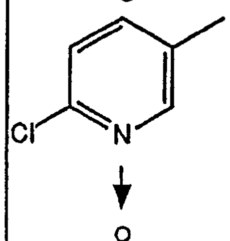 | $-CH_3$ | H | $-C_2H_5$ | $-CCl_2CF_3$ | |

| Verb. Nr | A | $R_1$ | $R_3$ | $R_4$ | $R_5$ | phys. Daten |
|---|---|---|---|---|---|---|
| 1.214 | 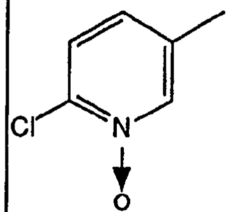 | H | $CH_3$ | $-CH_3$ | $-CCl_2CF_3$ | |
| 1.215 | 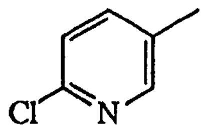 | H | $-CH_3$ | $-CH_3$ | $-CF_2CF_3$ | Smp. 105-108°C |
| 1.216 | 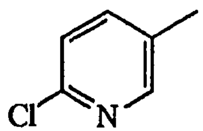 | $-C_2H_5$ | H | $-CH_3$ | $-CF_2CF_3$ | |
| 1.217 | 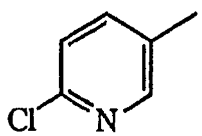 | 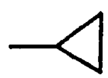 | H | $-CH_3$ | $-CF_2CF_3$ | |

| Verb. Nr | A | $R_1$ | $R_3$ | $R_4$ | $R_5$ | phys. Daten |
|---|---|---|---|---|---|---|
| 1.218 | 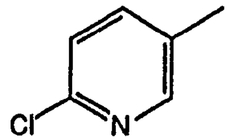 | $-CH_3$ | H | $-C_2H_5$ | $CF_2CF_3$ | |
| 1.219 | 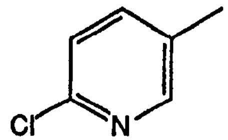 | $-CH_3$ | H | 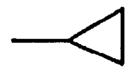 | $CF_2CF_3$ | |
| 1.220 | 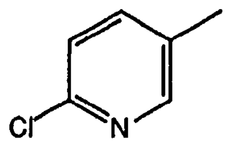 | $-C_2H_5$ | H | $-C_2H_5$ | $CF_2CF_3$ | |
| 1.221 | 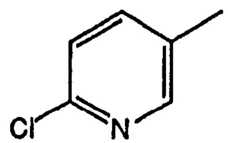 | $-C_2H_5$ | H | 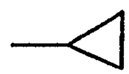 | $CF_2CF_3$ | |
| 1.222 | 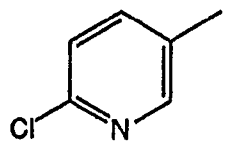 | 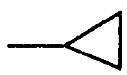 | H | $-C_2H_5$ | $CF_2CF_3$ | |

| Verb. Nr | A | $R_1$ | $R_3$ | $R_4$ | $R_5$ | phys. Daten |
|---|---|---|---|---|---|---|
| 1.223 | 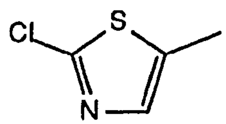 | $-C_2H_5$ | H | $-CH_3$ | $CF_2CF_3$ | |
| 1.224 | 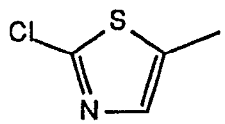 | 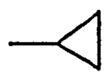 | H | $-CH_3$ | $CF_2CF_3$ | |
| 1.225 | 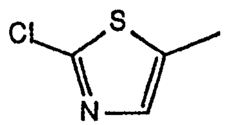 | $-CH_3$ | H | $-C_2H_5$ | $CF_2CF_3$ | |
| 1.226 | 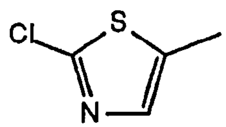 | $-CH_3$ | H | 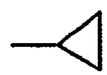 | $CF_2CF_3$ | |
| 1.227 | 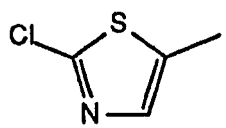 | $-C_2H_5$ | H | $-C_2H_5$ | $CF_2CF_3$ | |

| Verb. Nr | A | $R_1$ | $R_3$ | $R_4$ | $R_5$ | phys. Daten |
|---|---|---|---|---|---|---|
| 1.228 | 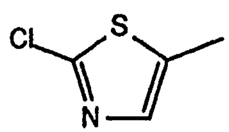 | $-C_2H_5$ | H | 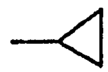 | $CF_2CF_3$ | |
| 1.229 | 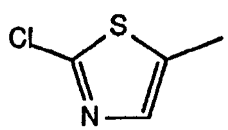 | 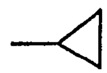 | H | $-C_2H_5$ | $CF_2CF_3$ | |
| 1.230 | 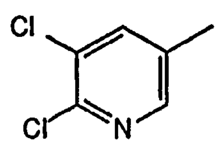 | $-CH_3$ | H | $-CH_3$ | $CF_2CF_3$ | |
| 1.231 | 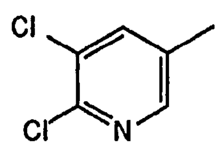 | $-C_2H_5$ | H | $-CH_3$ | $CF_2CF_3$ | |
| 1.232 | 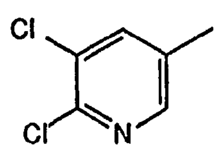 | 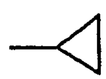 | H | $-CH_3$ | $CF_2CF_3$ | |

| Verb. Nr | A | $R_1$ | $R_3$ | $R_4$ | $R_5$ | phys. Daten |
|---|---|---|---|---|---|---|
| 1.233 | 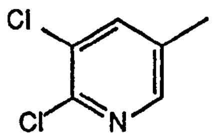 | $-CH_3$ | H | $-C_2H_5$ | $CF_2CF_3$ | |
| 1.234 | 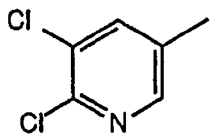 | $-CH_3$ | H | 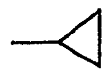 | $CF_2CF_3$ | |
| 1.235 | 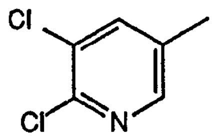 | $-C_2H_5$ | H | $-C_2H_5$ | $CF_2CF_3$ | |
| 1.236 | 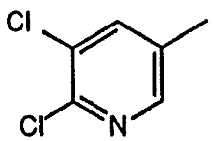 | $-C_2H_5$ | H | 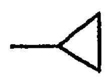 | $CF_2CF_3$ | |
| 1.237 | 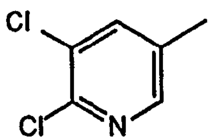 | 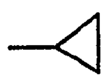 | H | $-C_2H_5$ | $CF_2CF_3$ | |
| 1.238 | 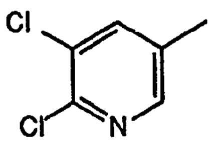 | H | $-CH_3$ | $-CH_3$ | $CF_2CF_3$ | |

| Verb. Nr | A | $R_1$ | $R_3$ | $R_4$ | $R_5$ | phys. Daten |
|---|---|---|---|---|---|---|
| 1.239 | 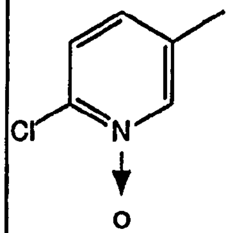 | $-CH_3$ | H | $-CH_3$ | $CF_2CF_3$ | |
| 1.240 | 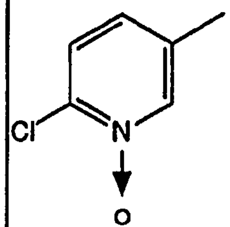 | $-C_2H_5$ | H | $-CH_3$ | $CF_2CF_3$ | |
| 1.241 | 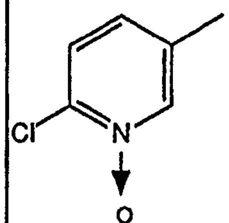 | 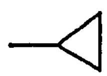 | H | $CH_3$ | $CF_2CF_3$ | |
| 1.242 | 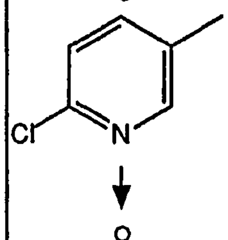 | $-CH_3$ | H | $-C_2H_5$ | $CF_2CF_3$ | |

| Verb. Nr | A | $R_1$ | $R_3$ | $R_4$ | $R_5$ | phys. Daten |
|---|---|---|---|---|---|---|
| 1.243 | 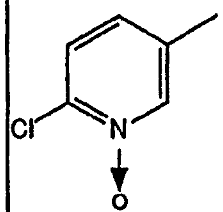 | H | $CH_3$ | $-CH_3$ | $CF_2CF_3$ | |

Beispiel 4: (Formulierungen - % = Gewichtsprozent)

| Beispiel 4.1: Emulsions-Konzentrate | a) | b) | c) |
|---|---|---|---|
| Wirkstoff gemäss Tabelle 1 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel 4.2: Lösungen | a) | b) | c) | d) |
|---|---|---|---|---|
| Wirkstoff gemäss Tabelle 1 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel 4.3: Granulate | a) | b) |
|---|---|---|
| Wirkstoff gemäss Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| Beispiel 4.4: Stäubemittel | a) | b) |
|---|---|---|
| Wirkstoff gemäss Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel 4.5: Spritzpulver | a) | b) | c) |
|---|---|---|---|
| Wirkstoff gemäss Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Beispiel 4.6: Emulsions-Konzentrat

| | |
|---|---|
| Wirkstoff gemäss Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel 4.7: Stäubemittel | a) | b) |
|---|---|---|
| Wirkstoff gemäss Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel 4.8: Extruder-Granulat

| | |
|---|---|
| Wirkstoff gemäss Tabelle 1 | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

Beispiel 4.9: Umhüllungs-Granulat

| | |
|---|---|
| Wirkstoff gemäss Tabelle 1 | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

Beispiel 4.10: Suspensions-Konzentrat

| | |
|---|---|
| Wirkstoff gemäss Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 5: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Nilaparvata lugens in diesem Test. Insbesondere die Verbindungen 1.001, 1.008, 1.009, 1.010, 1.011, 1.012, 1.015, 1.022, 1.024, 1.025, 1.028, 1.039, 1.081, 1.085, 1.101, 1.102, 1.103, 1.149, 1.153, 1.157, 1.165, 1.169, 1.183 und 1.189 zeigen eine Wirkung von über 80 %.

Beispiel 6: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Nephotettix cincticeps in diesem Test. Insbesondere die Verbindungen 1.001, 1.008, 1.009, 1.010, 1.011, 1.012, 1.015, 1.022, 1.024, 1.025, 1.028, 1.039, 1.081, 1.085, 1.101, 1.102, 1.103, 1.149, 1.153, 1.157, 1.165, 1.169, 1.183 und 1.184 zeigen eine Wirkung von über 80 %.

Beispiel 7: Wirkung gegen Spodoptera littoralis Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des dritten Stadiums von Spodoptera littoralis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Die Verbindungen der Tabelle 1 zeigen eine gute Wirkung gegen Spodoptera littoralis in diesem Test. Insbesondere die Verbindung 1.015 zeigt eine Wirkung von über 80 %.

Beispiel 8: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Aphis craccivora in diesem Test. Insbesondere die Verbindungen 1.008, 1.010, 1.011, 1.012, 1.015, 1.022, 1.028, 1.081, 1.084, 1.085, 1.101, 1.157, 1.165 und 1.169 zeigen eine Wirkung von über 80 %.

Beispiel 9: Systemische Wirkung gegen Nilaparvata lugens

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Nilaparvata lugens in diesem Test. Insbesondere die Verbindungen 1.001, 1.008, 1.010, 1.011, 1.012, 1.015, 1.022, 1.024, 1.025, 1.028, 1.039, 1.081, 1.084, 1.085, 1.101, 1.102, 1.103, 1.149, 1.153, 1.157, 1.165, 1.169, 1.183 und 1.184 zeigen selbst bei einer Konzentration von 12,5 ppm eine Wirkung über 80 %.

Beispiel 10: Systemische Wirkung gegen Nephotettix cincticeps

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Nephotettix cincticeps in diesem Test. Insbesondere die Verbindungen 1.008, 1.010, 1.011, 1.012, 1.015, 1.022, 1.024, 1.025, 1.028, 1.039, 1.081, 1.084, 1.085, 1.101, 1.102, 1.103, 1.149, 1.153, 1.157, 1.165, 1.169, 1.183 und 1.184 zeigen eine Wirkung von über 80 %.

Beispiel 11: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Myzus persicae in diesem Test. Insbesondere die Verbindungen 1.008, 1.010, 1.011, 1.012, 1.015, 1.028, 1.081, 1.084, 1.085, 1.101, 1.149, 1.153, 1.157, 1.165 und 1.169 zeigen eine Wirkung von über 80 %.

Beispiel 12: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten von Anthonomus grandis besiedelt und in einen Plastikbehälter gegeben. 3 Tage später erfolgt die Auswertung.

Aus dem Vergleich der Anzahl toter Käfer und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Die Verbindungen der Tabelle 1 zeigen eine gute Wirkung gegen Anthonomus grandis in diesem Test. Insbesondere die Verbindungen 1.008, 1.010, 1.011, 1.012, 1.015, 1.022, 1.028, 1.101, 1.149 und 1.153 zeigen eine Wirkung von über 80 %.

Beispiel 13: Wirkung gegen Bemisia tabaci

Buschbohnen-Pflanzen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindlichen Nymphen mit einer wässrigen Emulsions-Spritzbrühe der zu prüfenden Wirkstoffe (Konzentration 400 ppm) behandelt. Die Auswertung erfolgt 14 Tage nach der Wirkstoffapplikation auf %-Schlupf im Vergleich zu den unbehandelten Kontrollansätzen.

Die Verbindungen gemäss Tabelle 1 zeigen in diesem Test gute Wirkung gegen Bemisia tabaci. Insbesondere die Verbindungen 1.011, 1.012, 1.015, 1.081, 1.084, 1.085, 1.101 und 1,102 zeigen eine Wirkung von über 80 %.

Beispiel 14: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Diabrotica balteata in diesem Test. Insbesondere die Verbindungen 1.010, 1.012 und 1.015 zeigen eine Wirkung von über 80 %.

Beispiel 15: Wirkung gegen Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Die Verbindungen der Tabelle 1 zeigen eine gute Wirkung gegen Heliothis virescens in diesem Test. Insbesondere die Verbindungen 1.010, 1.011 und 1.015 zeigen eine Wirkung von über 80 %.

Beispiel 16: Wirkung gegen Crocidolomia binotalis Raupen

Junge Kohlpflanzen werden mit einer wässrigen Emulsions-Spritzbrühe, die 400 ppm des Wirkstoffes enthält, besprüht. Nach dem Antrocknen des Spritzbelages werden die Kohlpflanzen mit 10 Raupen des dritten Stadiums von Crocidolomia binotalis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Die Verbindungen der Tabelle 1 zeigen eine gute Wirkung in diesem Test. Insbesondere die Verbindung 1.015 zeigt eine Wirkung von über 80 %.

Beispiel 17: Wirkung gegen Myzus persicae

Erbsenkeimlinge werden mit Myzus persicae infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung in diesem Test. Insbesondere die Verbindungen 1.008, 1.010, 1.012, 1.015, 1.022 und 1.157 zeigen eine Wirkung von über 80 %.

## Patentansprüche

1. Verbindung der Formel I

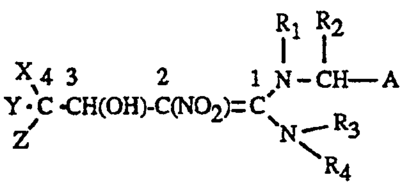

(I)

worin
$R_1$ Wasserstoff, $C_1$-$C_6$-Alkyl oder $C_3$-$C_6$-Cycloalkyl,
$R_2$ Wasserstoff oder $C_1$-$C_6$-Alkyl,
$R_3$ Wasserstoff, $C_1$-$C_6$-Alkyl oder $C_3$-$C_6$-Cycloalkyl,
$R_4$ Wasserstoff, $C_1$-$C_6$-Alkyl, $C_3$-$C_6$-Cycloalkyl oder einen Rest -$CH_2$-B, oder $R_4$ und $R_4$ gemeinsam -$(CH_2)_4$- oder -$(CH_2)_5$-;
X Halogen oder $C_1$-$C_6$-Haloalkyl;
Y und Z Halogen;
A einen unsubstituierten oder ein- bis vierfach substituierten aromatischen oder nicht-aromatischen, monocyclischen oder bicyclischen heterocyclischen Rest, worin ein oder zwei Substituenten der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten der Gruppe $C_1$-$C_3$-Alkyl, $C_1$-$C_3$-Alkoxy und Halogen angehören;

B Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl bedeuten, sowie deren Salze mit anorganischen Säuren; wobei nur einer der Reste $R_1$, $R_3$ und $R_4$ für Wasserstoff stehen kann.

2. Verbindung der Formel I gemäss Anspruch 1, dadurch gekennzeichnet, dass der heterocyclische Rest A ungesättigt ist und über eines seiner Ring-Kohlenstoffatome an den Rest der Verbindung der Formel I gebunden ist.

3. Verbindung der Formel I gemäss Anspruch 2, dadurch gekennzeichnet, dass der heterocyclische Rest A mindestens ein Stickstoffatom enthält.

4. Verbindung der Formel I gemäss Anspruch 2, dadurch gekennzeichnet, dass der heterocyclische Rest A ein bis drei Heteroatome aus der Reihe Sauerstoff, Schwefel und Stickstoff enthält, von denen höchstens eines Sauerstoff- oder Schwefelatom ist.

5. Verbindung der Formel I gemäss Anspruch 2, dadurch gekennzeichnet, dass der heterocyclische Rest A einen Grundkörper aus der Gruppe

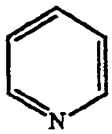
,
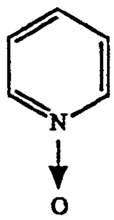
,
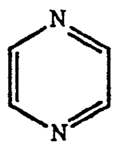
,
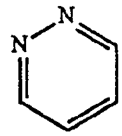
,
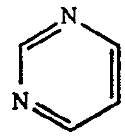
,
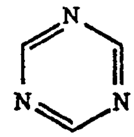
,
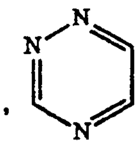
,

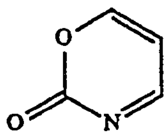
,
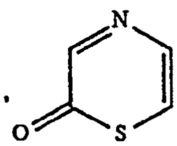
,
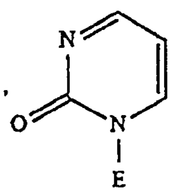
,
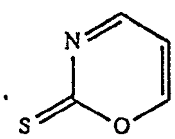
,
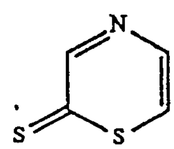
,
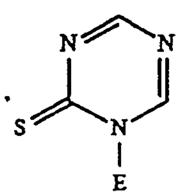
,

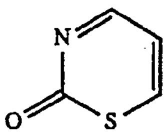 , 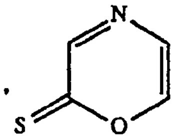 , 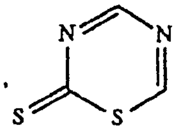 , 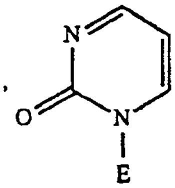 , 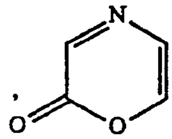 ,

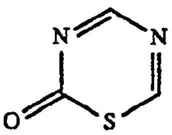 , 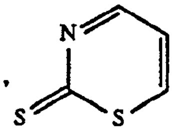 , 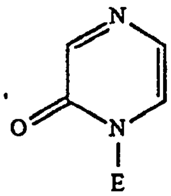 , 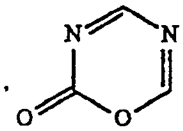 , 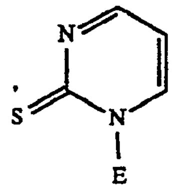 , 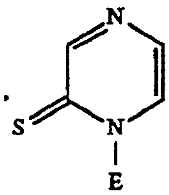 ,

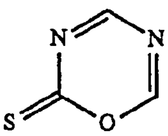 , 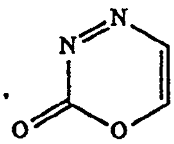 , 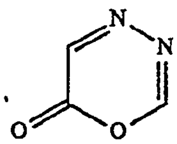 , 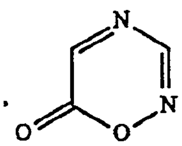 , 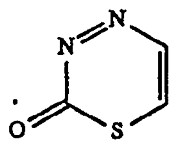 ,

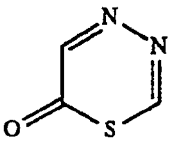 , 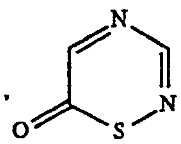 , 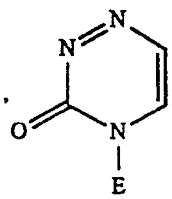 , 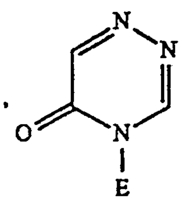 , 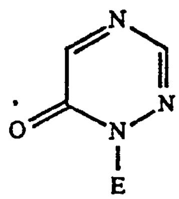 ,

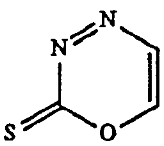 , 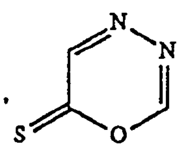 , 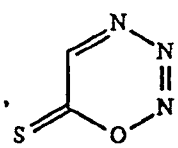 , 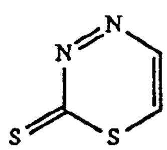 , 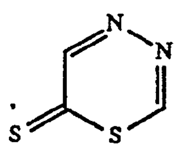 ,

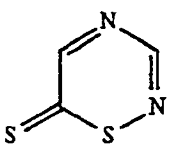 , 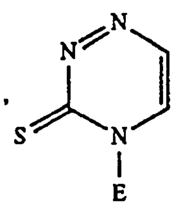 , 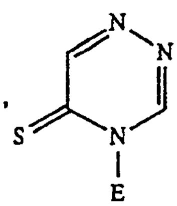 , 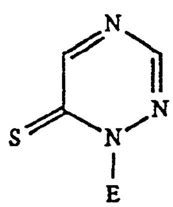 , 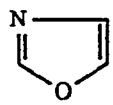 ,

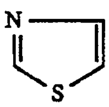 , 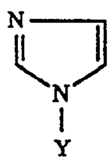 , 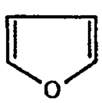 , 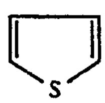 ,

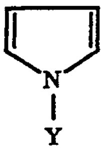 , 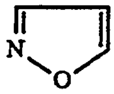 , 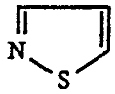 , 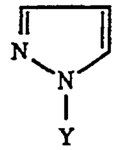 ,

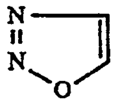 , 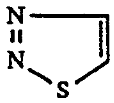 , 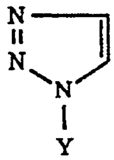 , 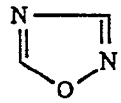 , 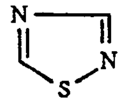 ,

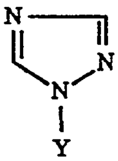 , 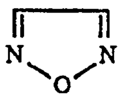 , 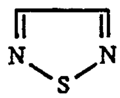 , 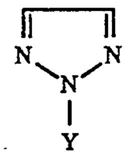 , 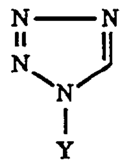 ,

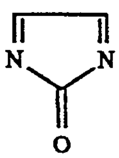 , 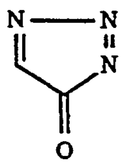 , 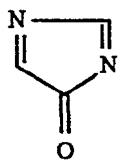 , 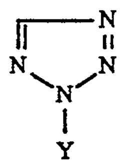 , 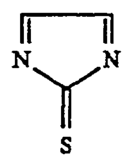 ,

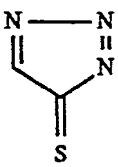 , 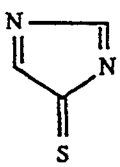 , 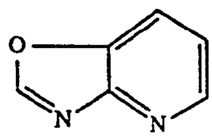 , 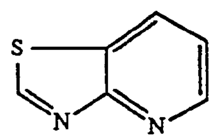 , 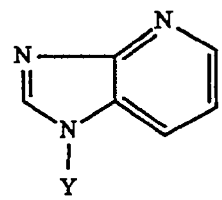 ,

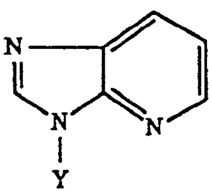 , 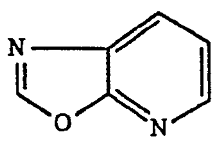 , 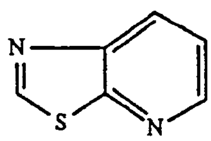 , 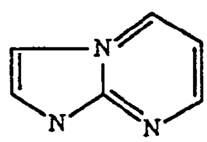 ,

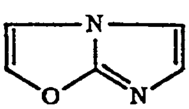 , 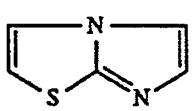 , 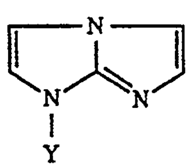 .

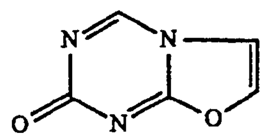 , 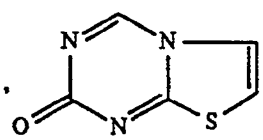 , 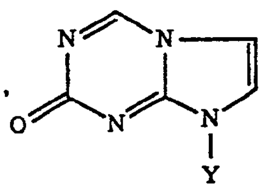 , 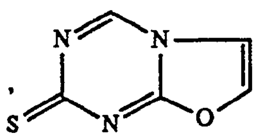 ,

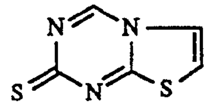 und 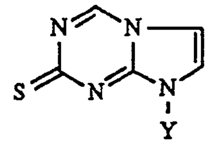

darstellt, welcher unsubstituiert ist oder je nach den Substitutionsmöglichkeiten des betreffenden Ringsystems bis zu vier der im Anspruch 1 definierten Substituenten trägt, wobei E für $C_1$-$C_3$-Alkyl und Y für Wasserstoff, $C_1$-$C_3$-Alkyl oder Cyclopropyl stehen.

6. Verbindung der Formel I gemäss einem der Ansprüche 1 bis 5, dadurch gekennzeichnet, dass die heterocyclischen Reste A unsubstituiert sind oder ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy tragen.

7. Verbindung der Formel I gemäss Anspruch 6, dadurch gekennzeichnet, dass die heterocyclischen Reste A Pyridylreste oder Thiazolylreste sind.

8. Verbindung der Formel I gemäss Anspruch 7, dadurch gekennzeichnet, dass A für 3-Pyridyl, 2-Halogenpyrid-5-yl, 2,3-Dihalogenpyrid-5-yl oder 2-Halogenthiazol-4-yl, 1-Oxopyrid-3-yl, 1-Oxo-2-halogenpyrid-5-yl oder 1-Oxo-2,3-dihalogenpyrid-5-yl steht.

9. Verbindung der Formel I gemäss Anspruch 1, dadurch gekennzeichnet, dass der Rest B für einen Phenyl-, Pyridyl- oder Thiazolylrest steht, welcher unsubstituiert oder durch ein bis zwei Reste aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiert ist.

10. Verbindung der Formel I gemäss Anspruch 1, dadurch gekennzeichnet, dass $R_1$ und $R_3$ Wasserstoff, Methyl, Äthyl oder Cyclopropyl bedeuten.

11. Verbindung der Formel I gemäss Anspruch 1, dadurch gekennzeichnet, dass $R_2$ Wasserstoff bedeutet.

12. Verbindung der Formel I gemäss Anspruch 1, dadurch gekennzeichnet, dass $R_4$ Wasserstoff oder Methyl bedeutet.

13. Verbindung der Formel I gemäss Anspruch 1, dadurch gekennzeichnet, dass $R_1$ und $R_3$ unabhängig voneinander Wasserstoff, Methyl, Äthyl oder Cyclopropyl, $R_2$ Wasserstoff, $R_4$ Wasserstoff oder Methyl, A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiertes Pyridyl, 1-Oxopyridyl oder Thiazolyl bedeuten.

14. Verbindung der Formel I gemäss einem der Ansprüche 1 bis 13, dadurch gekennzeichnet, dass X Fluor, Chlor, Brom oder $C_1$-$C_3$-Haloalkyl mit 1 bis 7 Fluoratomen und Y und Z unabhängig voneinander Fluor, Chlor oder Brom bedeuten.

15. Verbindung der Formel I gemäss einem der Ansprüche 1 bis 13, dadurch gekennzeichnet, dass X, Y und Z unabhängig voneinander Fluor, Chlor oder Brom bedeuten.

16. Verbindung der Formel I gemäss Anspruch 15, dadurch gekennzeichnet, dass X, Y und Z Chlor bedeuten.

17. Verbindung der Formel I gemäss Anspruch 13, dadurch gekennzeichnet, dass $R_1$ und $R_3$ Methyl;
$R_2$ und $R_4$ Wasserstoff; und

X, Y und Z unabhängig voneinander Fluor, Chlor oder Brom bedeuten.

18. Verbindung der Formel I gemäss einem der Ansprüche 13 bis 17, dadurch gekennzeichnet, dass A Pyridyl oder durch ein oder zwei Chloratome substituiertes Pyridyl bedeutet.

19. Verbindung gemäss Anspruch 15, dadurch gekennzeichnet, dass X Fluor oder Brom, Y und Z Halogen bedeuten.

20. Verbindung gemäss Anspruch 19, dadurch gekennzeichnet, dass X Fluor, Y und Z Fluor oder Chlor bedeuten.

21. Verbindung gemäss Anspruch 14, dadurch gekennzeichnet, dass X $C_1$-$C_6$-Halogenalkyl mit 4 bis 13 Halogenatomen bedeutet.

22. Verbindung gemäss Anspruch 21, dadurch gekennzeichnet, dass X $C_1$-$C_6$-Halogenalkyl mit 2 bis 13 Halogenatomen bedeutet.

23. Verbindung gemäss Anspruch 14, dadurch gekennzeichnet, dass X Trifluormethyl bedeutet.

24. Verbindung gemäss Anspruch 14, dadurch gekennzeichnet, dass X einen Rest $-C_2F_5$ oder $-C_3F_7$ bedeutet.

25. Verbindung der Formel I gemäss einem der Ansprüche 13 bis 24, dadurch gekennzeichnet, dass A Thiazolyl oder durch ein Chloratom substituiertes Thiazolyl, vorzugsweise 1,3-Thiazol-5-yl, bedeutet.

26. Verbindung gemäss Anspruch 16 der Formel

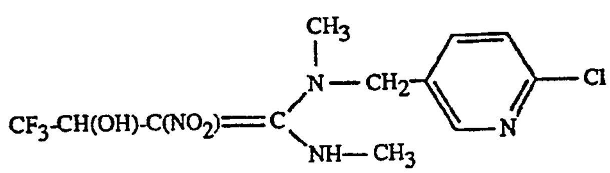

27. Verbindung gemäss Anspruch 16 der Formel

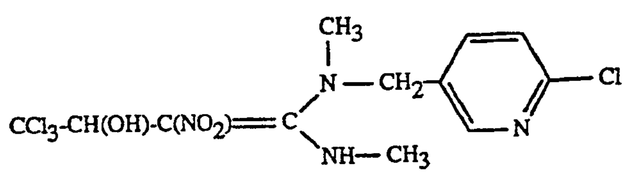

28. Verbindung gemäss Anspruch 16 der Formel

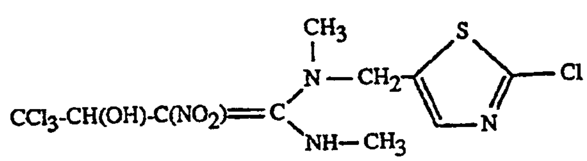

29. Verbindung gemäss Anspruch 16 der Formel

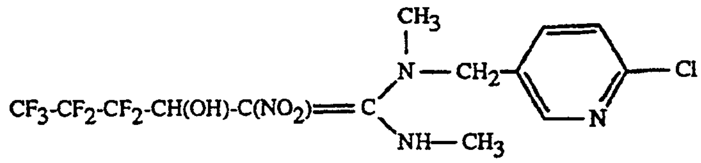

**30.** Verbindung gemäss Anspruch 16 der Formel

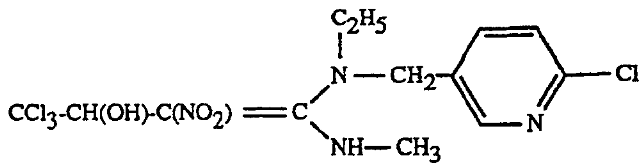

**31.** Verbindung der Formel I gemäss Anspruch 13, ausgewählt aus der Gruppe

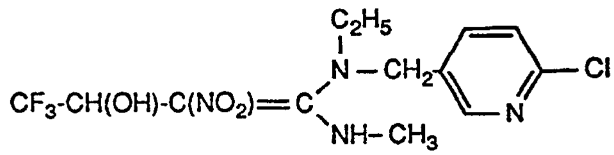

,

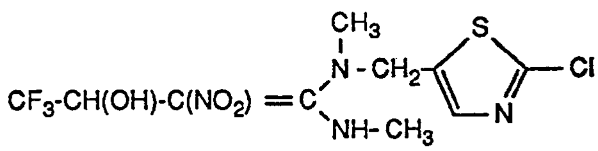

,

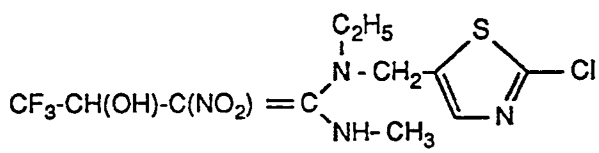

,

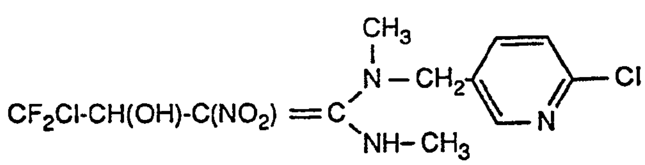

,

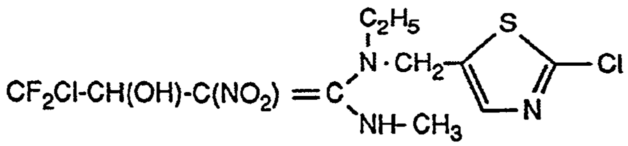
,

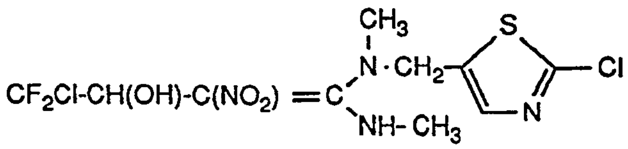
,

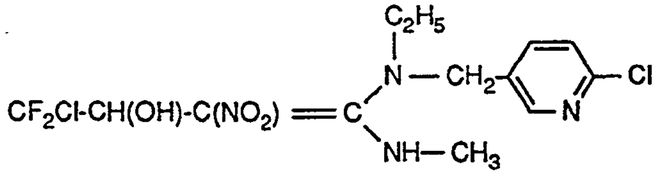
,

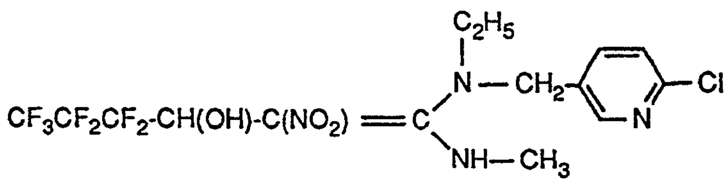
,

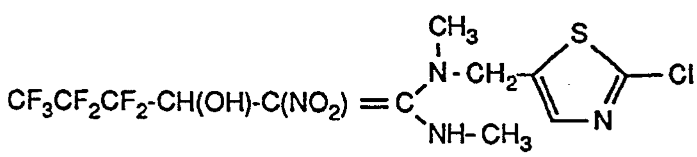
,

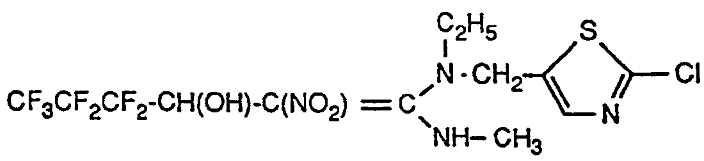
,

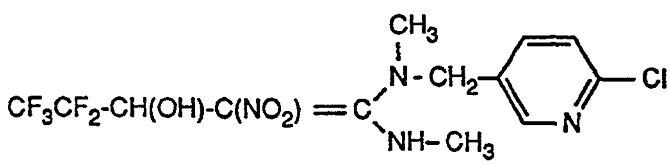
,

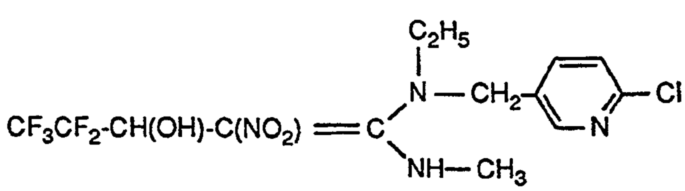
,

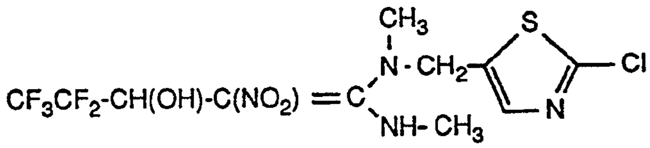 ,

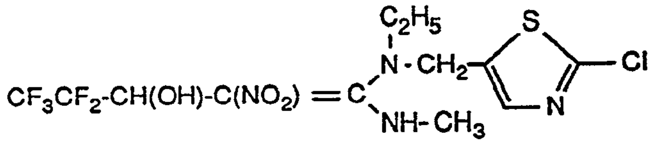 ,

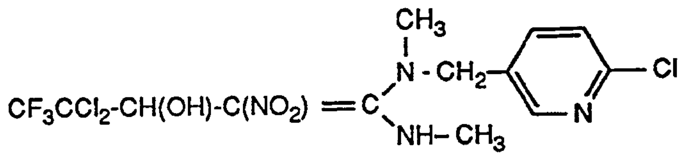 ,

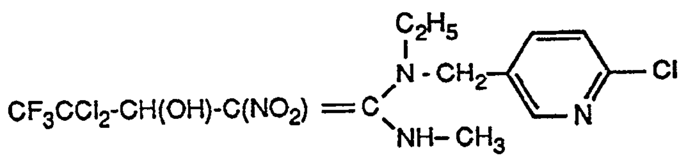 ,

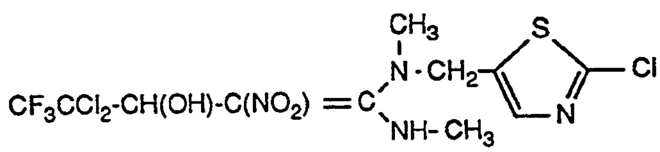 und

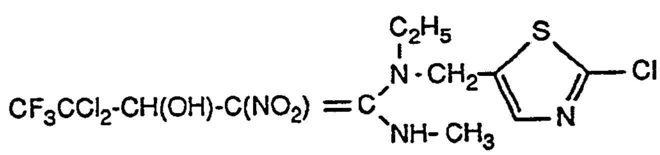 .

**32.** Verfahren zur Herstellung einer Verbindung der Formel I gemäss Anspruch 1

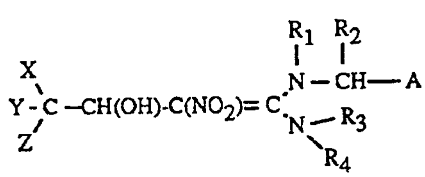 (I)

dadurch gekennzeichnet, dass man eine Verbindung der Formel II

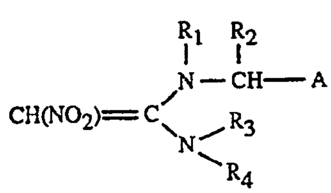 (II)

mit einer Verbindung der Formel III

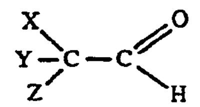

(III)

unter pH-neutralen Reaktionsbedingungen umsetzt, wobei $R_1$ bis $R_4$, A, X, Y und Z die in Anspruch 1 angegebenen Bedeutungen haben.

33. Mittel zur Bekämpfung von Schädlingen, welches neben inerten Zusätzen und Formulierungshilfsmitteln als aktive Komponente eine pestizid wirksame Menge einer Verbindung der Formel I gemäss Anspruch 1 enthält.

34. Verwendung einer Verbindung gemäss Anspruch 1 zur Bekämpfung von Insekten und Vertretern der Ordnung Akarina an Tieren und Pflanzen.

35. Verwendung gemäss Anspruch 34 zur Bekämpfung von pflanzenschädigenden Insekten.

36. Verwendung gemäss Anspruch 35 zur Bekämpfung von pflanzenschädigenden saugenden Insekten.

37. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss Anspruch 1 oder mit einem Mittel enthaltend neben Zusatz- und Trägerstoffen eine pestizid wirksame Menge dieser Verbindung, in Kontakt bringt oder behandelt.

38. Verfahren gemäss Anspruch 37 zur Bekämpfung von pflanzenschädigenden Insekten.

39. Verfahren gemäss Anspruch 38 zur Bekämpfung von pflanzenschädigenden saugenden Insekten.

**Patentansprüche Für folgenden Vertragsstaat: ES**

1. Verfahren zur Herstellung einer Verbindung der Formel I

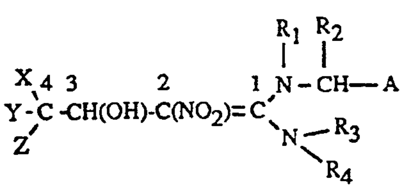

(I)

worin
$R_1$ Wasserstoff, $C_1$-$C_6$-Alkyl oder $C_3$-$C_6$-Cycloalkyl,
$R_2$ Wasserstoff oder $C_1$-$C_6$-Alkyl,
$R_3$ Wasserstoff, $C_1$-$C_6$-Alkyl oder $C_3$-$C_6$-Cycloalkyl,
$R_4$ Wasserstoff, $C_1$-$C_6$-Alkyl, $C_3$-$C_6$-Cycloalkyl oder einen Rest $-CH_2$-B, oder
$R_3$ und $R_4$ gemeinsam $-(CH_2)_4-$ oder $-(CH_2)_5-$;
X Halogen oder $C_1$-$C_6$-Haloalkyl;
Y und Z Halogen;
A einen unsubstituierten oder ein- bis vierfach substituierten aromatischen oder nicht-aromatischen, monocyclischen oder bicyclischen heterocyclischen Rest, worin ein oder zwei Substituenten der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten der Gruppe $C_1$-$C_3$-Alkyl, $C_1$-$C_3$-Alkoxy und Halogen angehören;

B Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-

$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl bedeuten, sowie deren Salze mit anorganischen Säuren; wobei nur einer der Reste $R_1$, $R_3$ und $R_4$ für Wasserstoff stehen kann
dadurch gekennzeichnet, dass man eine Verbindung der Formel II

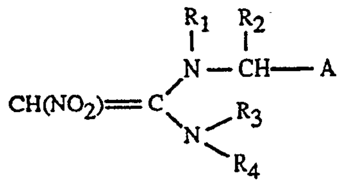

(II)

mit einer Verbindung der Formel III

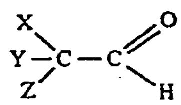

(III)

unter pH-neutralen Reaktionsbedingungen umsetzt, wobei $R_1$ bis $R_4$, A, X, Y und Z die oben angegebenen Bedeutungen haben.

2. Verfahren zur Herstellung einer Verbindung der Formel I gemäss Anspruch 1, dadurch gekennzeichnet, dass der heterocyclische Rest A ungesättigt ist und über eines seiner Ring-Kohlenstoffatome an den Rest der Verbindung der Formel I gebunden ist.

3. Verfahren zur Herstellung einer Verbindung der Formel I gemäss Anspruch 2, dadurch gekennzeichnet, dass der heterocyclische Rest A mindestens ein Stickstoffatom enthält.

4. Verfahren zur Herstellung einer Verbindung der Formel I gemäss Anspruch 2, dadurch gekennzeichnet, dass der heterocyclische Rest A ein bis drei Heteroatome aus der Reihe Sauerstoff, Schwefel und Stickstoff enthält, von denen höchstens eines Sauerstoff- oder Schwefelatom ist.

5. Verfahren zur Herstellung einer Verbindung der Formel I gemäss Anspruch 2, dadurch gekennzeichnet, dass der heterocyclische Rest A einen Grundkörper aus der Gruppe

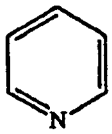 , 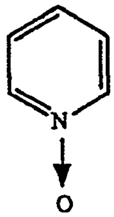 , 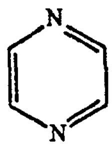 , 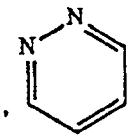 , 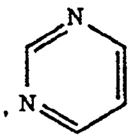 , 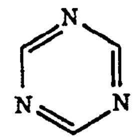 , 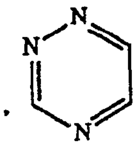 .

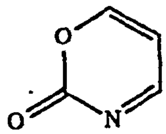 , 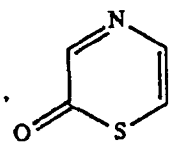 , 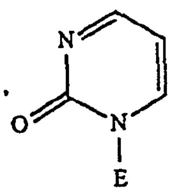 , 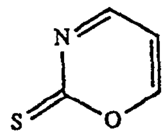 , 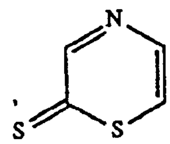 , 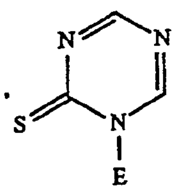 ,

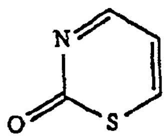 , 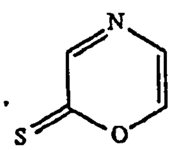 , 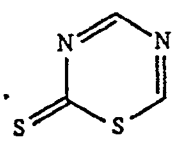 , 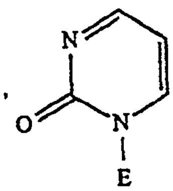 , 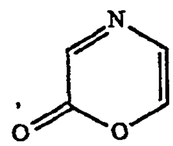 ,

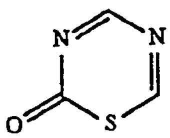 , 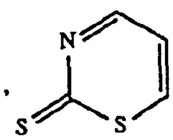 , 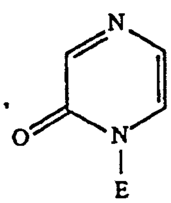 , 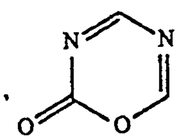 , 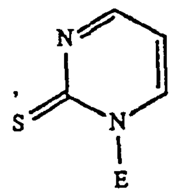 , 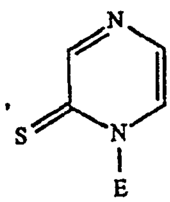 ,

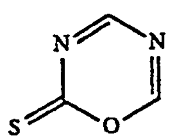 , 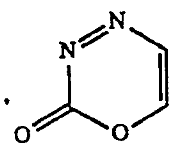 , 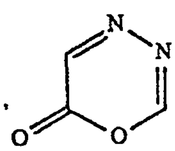 , 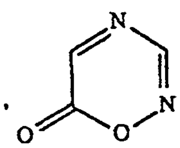 , 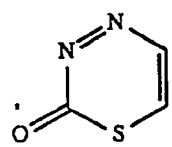 ,

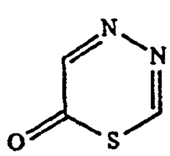 , 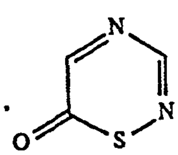 , 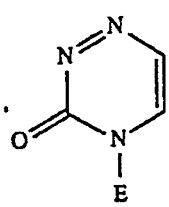 , 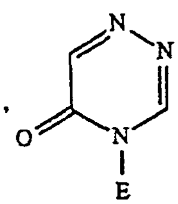 , 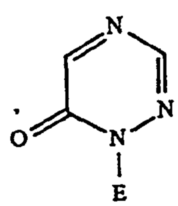 .

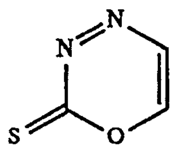 , 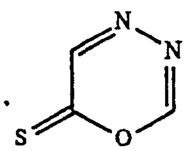 , 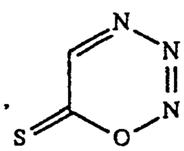 , 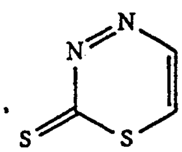 , 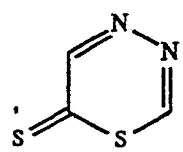 ,

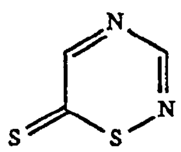 , 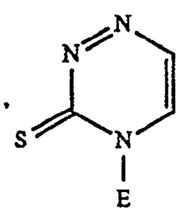 , 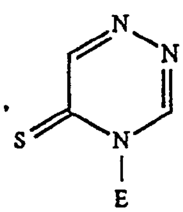 , 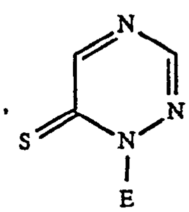 , 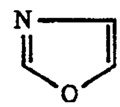 ,

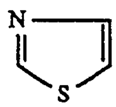 , 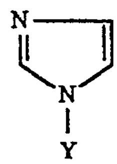 , 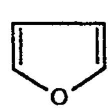 , 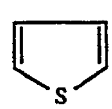 ,

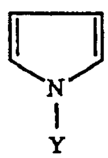 , 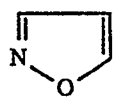 , 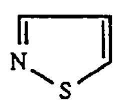 , 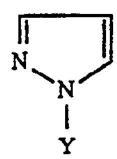 ,

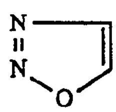 , 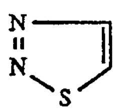 , 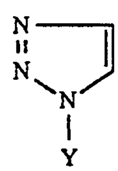 , 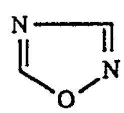 , 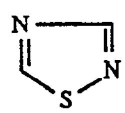 ,

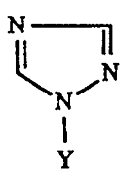 , 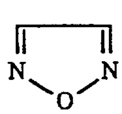 , 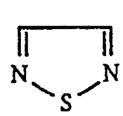 , 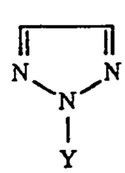 , 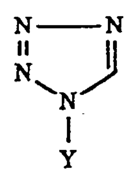 ,

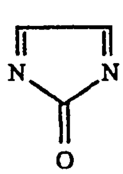 , 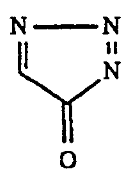 , 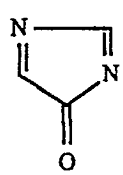 , 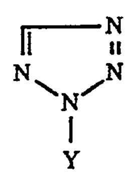 , 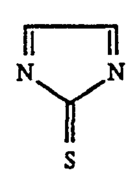 .

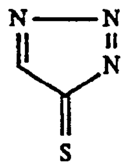 , 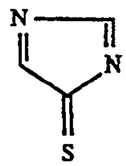 , 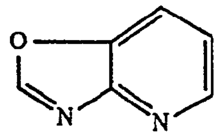 , 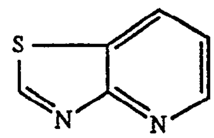 , 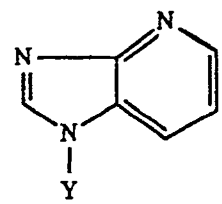 ,

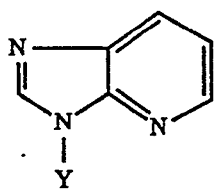 , 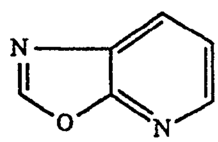 , 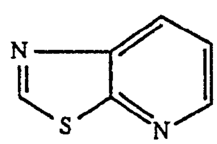 , 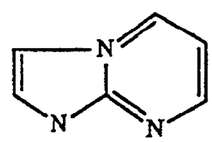 ,

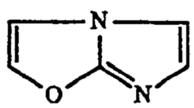 , 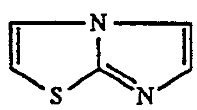 , 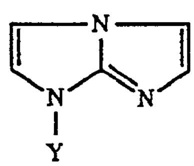 ,

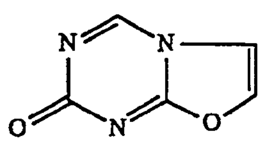 , 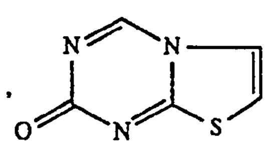 , 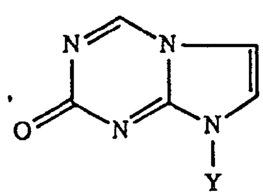 , 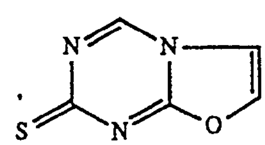 ,

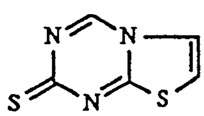 und 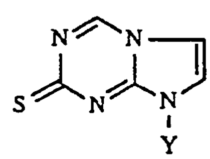

darstellt, welcher unsubstituiert ist oder je nach den Substitutionsmöglichkeiten des betreffenden Ringsystems bis zu vier der im Anspruch 1 definierten Substituenten trägt, wobei E für $C_1$-$C_3$-Alkyl und Y für Wasserstoff, $C_1$-$C_3$-Alkyl oder Cyclopropyl stehen.

6. Verfahren zur Herstellung einer Verbindung der Formel I gemäss einem der Ansprüche 1 bis 5, dadurch gekennzeichnet, dass die heterocyclischen Reste A unsubstituiert sind oder ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy tragen.

7. Verfahren zur Herstellung einer Verbindung der Formel I gemäss Anspruch 6, dadurch gekennzeichnet, dass die heterocyclischen Reste A Pyridylreste oder Thiazolylreste sind.

8. Verfahren zur Herstellung einer Verbindung der Formel I gemäss Anspruch 7, dadurch gekennzeichnet, dass A für 3-Pyridyl, 2-Halogenpyrid-5-yl, 2,3-Dihalogenpyrid-5-yl oder 2-Halogenthiazol-4-yl, 1-Oxopyrid-3-yl, 1-Oxo-2-halogenpyrid-5-yl oder 1-Oxo-2,3-dihalogenpyrid-5-yl steht.

9. Verfahren zur Herstellung einer Verbindung der Formel I gemäss Anspruch 1, dadurch gekennzeichnet, dass der Rest B für einen Phenyl-, Pyridyl- oder Thiazolylrest steht, welcher unsubstituiert oder durch ein bis zwei Reste aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiert ist.

10. Verfahren zur Herstellung einer Verbindung der Formel I gemäss Anspruch 1, dadurch gekennzeichnet, dass $R_1$ und $R_3$ Wasserstoff, Methyl, Äthyl oder Cyclopropyl bedeuten.

11. Verfahren zur Herstellung einer Verbindung der Formel I gemäss Anspruch 1, dadurch gekennzeichnet, dass $R_2$ Wasserstoff bedeutet.

12. Verfahren zur Herstellung einer Verbindung der Formel I gemäss Anspruch 1, dadurch gekennzeichnet, dass $R_4$ Wasserstoff oder Methyl bedeutet.

13. Verfahren zur Herstellung einer Verbindung der Formel I gemäss Anspruch 1, dadurch gekennzeichnet, dass $R_1$ und $R_3$ unabhängig voneinander Wasserstoff, Methyl, Äthyl oder Cyclopropyl, $R_2$ Wasserstoff, $R_4$ Wasserstoff oder Methyl, A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiertes Pyridyl, 1-Oxopyridyl oder Thiazolyl bedeuten.

14. Verfahren zur Herstellung einer Verbindung der Formel I gemäss einem der Ansprüche 1 bis 13, dadurch gekennzeichnet, dass X Fluor, Chlor, Brom oder $C_1$-$C_3$-Haloalkyl mit 1 bis 7 Fluoratomen und Y und Z unabhängig voneinander Fluor, Chlor oder Brom bedeuten.

15. Verfahren zur Herstellung einer Verbindung der Formel I gemäss einem der Ansprüche 1 bis 13, dadurch gekennzeichnet, dass X, Y und Z unabhängig voneinander Fluor, Chlor oder Brom bedeuten.

16. Verfahren zur Herstellung einer Verbindung der Formel I gemäss Anspruch 15, dadurch gekennzeichnet, dass X, Y und Z Chlor bedeuten.

17. Verfahren zur Herstellung einer Verbindung der Formel I gemäss Anspruch 13, dadurch gekennzeichnet, dass
$R_1$ und $R_3$ Methyl;
$R_2$ und $R_4$ Wasserstoff; und
X, Y und Z unabhängig voneinander Fluor, Chlor oder Brom bedeuten.

18. Verfahren zur Herstellung einer Verbindung der Formel I gemäss einem der Ansprüche 13 bis 17, dadurch gekennzeichnet, dass A Pyridyl oder durch ein oder zwei Chloratome substituiertes Pyridyl bedeutet.

19. Verfahren zur Herstellung einer Verbindung gemäss Anspruch 15, dadurch gekennzeichnet, dass X Fluor oder Brom, Y und Z Halogen bedeuten.

20. Verfahren zur Herstellung einer Verbindung gemäss Anspruch 19, dadurch gekennzeichnet, dass X Fluor, Y und Z Fluor oder Chlor bedeuten.

21. Verfahren zur Herstellung einer Verbindung gemäss Anspruch 14, dadurch gekennzeichnet, dass X $C_1$-$C_6$-Halogenalkyl mit 4 bis 13 Halogenatomen bedeutet.

22. Verfahren zur Herstellung einer Verbindung gemäss Anspruch 21, dadurch gekennzeichnet, dass X $C_1$-$C_6$-Halogenalkyl mit 2 bis 13 Halogenatomen bedeutet.

23. Verfahren zur Herstellung einer Verbindung gemäss Anspruch 14, dadurch gekennzeichnet, dass X Trifluormethyl bedeutet.

24. Verfahren zur Herstellung einer Verbindung gemäss Anspruch 14, dadurch gekennzeichnet, dass X einen Rest $-C_2F_5$ oder $-C_3F_7$, bedeutet.

25. Verfahren zur Herstellung einer Verbindung der Formel I gemäss einem der Ansprüche 13 bis 24, dadurch gekennzeichnet, dass A Thiazolyl oder durch ein Chloratom substituiertes Thiazolyl, vorzugsweise 1,3-Thiazol-5-yl, bedeutet.

26. Verfahren gemäss Anspruch 16 zur Herstellung der Verbindung der Formel

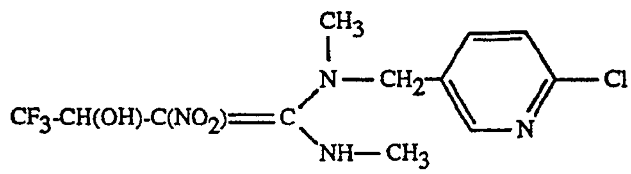

**27.** Verfahren gemäss Anspruch 16 zur Herstellung der Verbindung der Formel

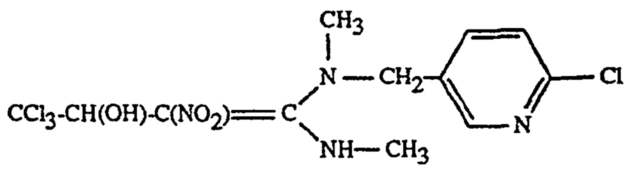

**28.** Verfahren gemäss Anspruch 16 zur Herstellung der Verbindung der Formel

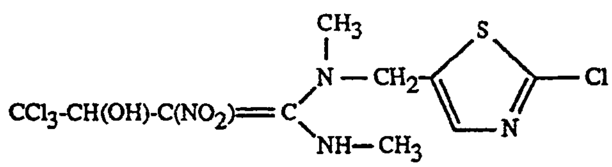

**29.** Verfahren gemäss Anspruch 16 zur Herstellung der Verbindung der Formel

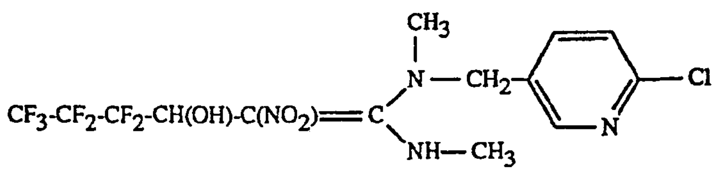

**30.** Verfahren gemäss Anspruch 16 zur Herstellung der Verbindung der Formel

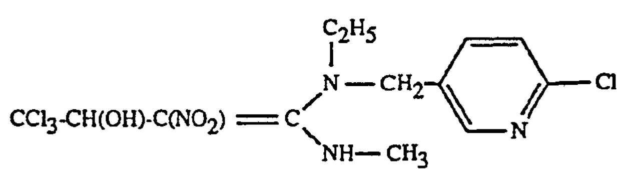

**31.** Verfahren der Formel I gemäss Anspruch 13, zur Herstellung einer Verbindung, ausgewählt aus der Gruppe

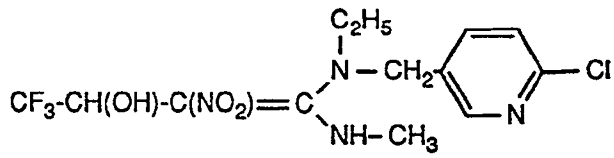 ,

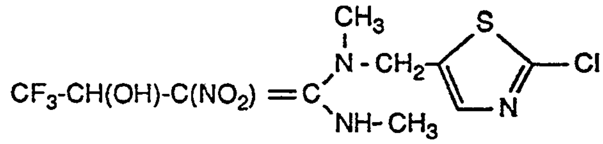 ,

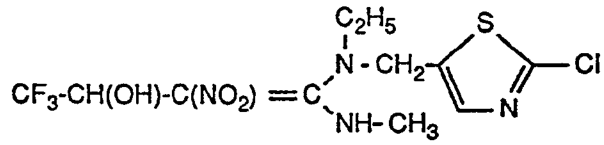 ,

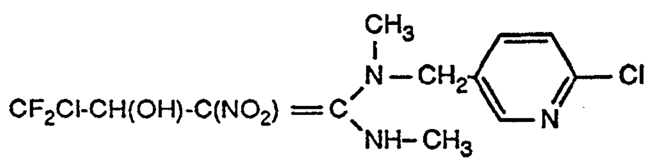 ,

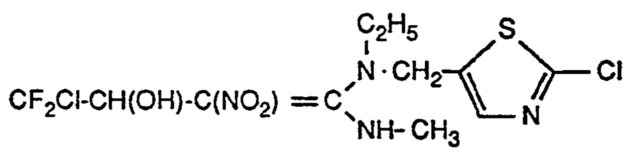 ,

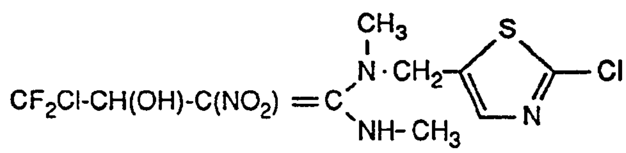 ,

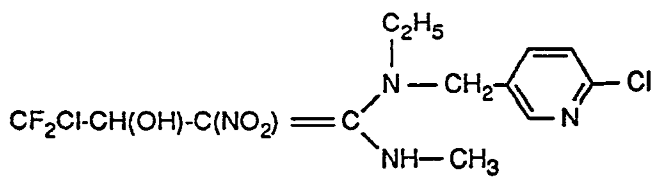 ,

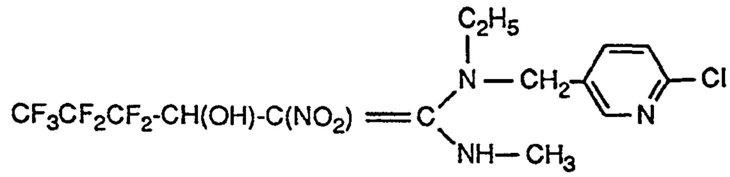,

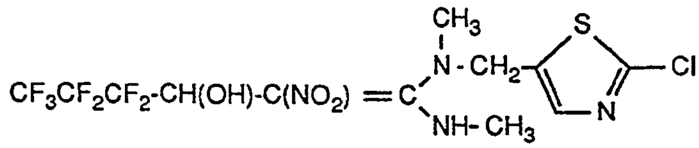,

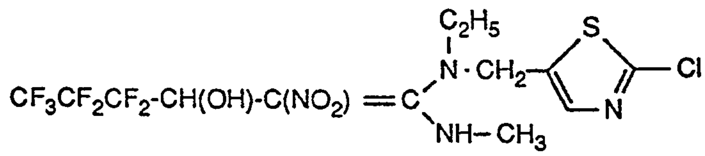,

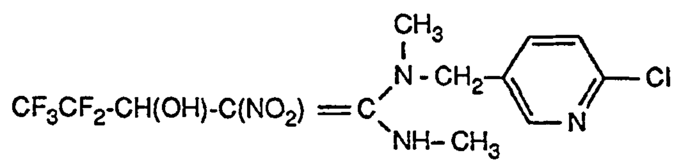,

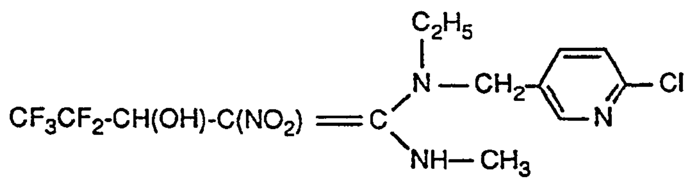,

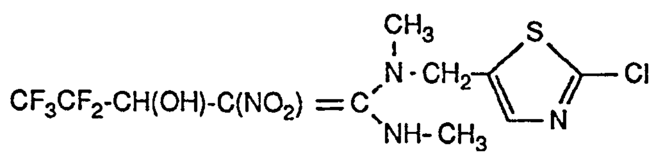,

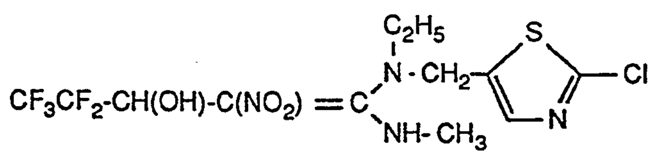,

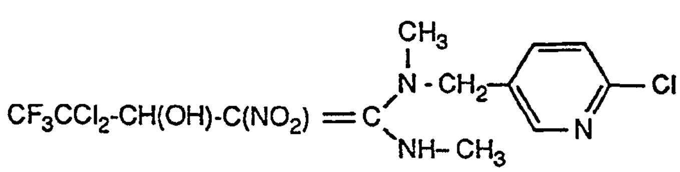,

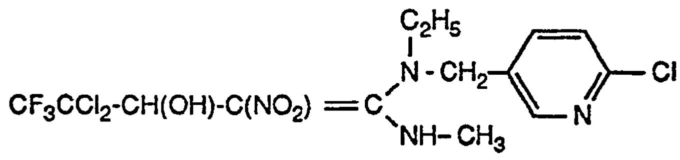 ,

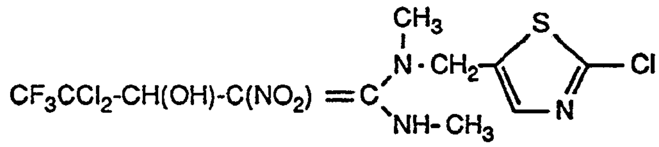 und

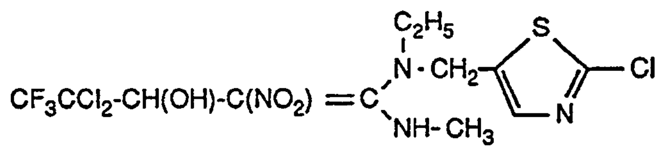 .

**32.** Mittel zur Bekämpfung von Schädlingen, welches neben inerten Zusätzen und Formulierungshilfsmitteln als aktive Komponente eine pestizid wirksame Menge einer Verbindung der Formel I gemäss Anspruch 1 enthält.

**33.** Verwendung einer Verbindung gemäss Anspruch 1 zur Bekämpfung von Insekten und Vertretern der Ordnung Akarina an Tieren und Pflanzen.

**34.** Verwendung gemäss Anspruch 33 zur Bekämpfung von pflanzenschädigenden Insekten.

**35.** Verwendung gemäss Anspruch 34 zur Bekämpfung von pflanzenschädigenden saugenden Insekten.

**36.** Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss Anspruch 1 oder mit einem Mittel enthaltend neben Zusatz- und Trägerstoffen eine pestizid wirksame Menge dieser Verbindung, in Kontakt bringt oder behandelt.

**37.** Verfahren gemäss Anspruch 36 zur Bekämpfung von pflanzenschädigenden Insekten.

**38.** Verfahren gemäss Anspruch 37 zur Bekämpfung von pflanzenschädigenden saugenden Insekten.